# EUROPEAN PATENT APPLICATION

(11) **EP 4 772 185 A1**
(43) Date of publication of application: **08.07.2026**
(21) Application number: 24860463.9
(22) Date of filing: 30.08.2024
(51) Int. Cl.: A61K 35/28, A61P 17/02, A61K 9/00

(54) **COMPOSITION COMPRISING MESENCHYMAL STEM CELL SPHEROID FOR TREATING SKIN WOUND OR SCAR**

(30) Priority: 01.09.2023 KR 20230116408
(71) Applicant: Cellincells Co., Ltd., Seoul 03080 (KR); Seoul National University R&DB Foundation, Seoul 08826 (KR)
(72) Inventor: CHO, Jaejin, Seoul 06288 (KR); PARK, Hyejung, Seoul 06291 (KR); PARK, Jie-Eun, Anyang-si, Gyeonggi-do 14061 (KR)
(74) Representative: Calysta NV
(86) International application number: PCT/KR2024/013054
(87) International publication number: WO 2025/048545

(57) **Abstract**

The present disclosure relates to a pharmaceutical composition comprising a therapeutically effective amount of mesenchymal stem cell spheroids which is topically applied to a target site or administered by subcutaneous or intradermal injection for treating skin wounds or scars. Administering mesenchymal stem cell spheroids to wound or scar areas promotes dermal and subcutaneous recovery within the skin, thereby enhancing skin regeneration, scar healing, recovery, and maintenance of skin volume.

## Description

### [Technical Field]

The present disclosure relates to a composition for treating skin wounds or scars, and more particularly, to a pharmaceutical composition or filler composition for treating skin wounds or scars, comprising mesenchymal stem cell spheroids as an active ingredient, which are applied topically or administered subcutaneously or intradermally.

### [Background Art]

The skin serves as the body's first line of defense against physical and environmental aggressors, and when damaged, the wound-healing process is activated, which may result in scarring. Ideally, wound healing should result in virtually scar-free skin, but some individuals may experience excessive fibrosis or atrophy, resulting in keloids, hypertrophic scars, or atrophic scars. Unlike keloid and hypertrophic scars, which involve tissue outgrowth, atrophic scars produce depressed scars. The topographic depression of atrophic scars is thought to be due to inadequate compensation of dermal collagen and connective tissue after injury. Atrophic scars may result from various causes, including scleroderma, stretch marks during pregnancy, acne, surgery, and accidents. Pathological scars, especially when present on visible areas of the body, may significantly reduce quality of life and lead to psychological stress. The present disclosure proposes a novel treatment method using self-assembling adipose-derived mesenchymal stem cells (MSCs), which may contribute to improving quality of life in scar treatment.

Various treatments have been used to improve the appearance of scars. These treatments include excision, lasers, and dermal filler injections. Hyaluronic acid (HA), collagen, poly-L-lactic acid, and calcium hydroxylapatite are commonly used dermal fillers. However, hyaluronic acid and collagen are short-term fillers, with short-term effects lasting only a few months. On the other hand, permanent fillers such as polymethylmethacrylate (PMMA) and silicone offer long-lasting effects but carry a higher risk of complications such as granulomas, silicone embolism syndrome, and nodule formation.

To overcome the limitations of these artificial fillers, new approaches are required, with stem cells emerging as a promising alternative. Mesenchymal stem cells (MSCs), adult stem cells, have been shown to have exceptional potential in promoting wound healing due to their self-renewal capacity, secretion of regenerative factors, and immunomodulatory properties. The cells may be obtained from various tissues, including bone marrow and adipose tissue. Currently, most cell therapy treatments are administered through injection or transplantation in single-cell form. Single-cell MSCs have low survival rates and very low engraftment rates after transplantation. Therefore, when using MSCs as a skin regeneration treatment, it is necessary to improve the survival rate and engraftment rate after transplantation, and in particular, the development of cell therapy products enriched with connective tissue components is crucial for the treatment of atrophic scars caused by the reduction of connective tissue.

Accordingly, to address the above issues, the inventors of the present disclosure established physical culture conditions optimized for skin regeneration and developed a composition containing stem cells rich in extracellular matrix and capable of engraftment and long-term volume maintenance, thereby completing the present disclosure. Furthermore, the inventors developed mesenchymal stem cell spheroids and confirmed that MSCs transplanted into the skin maintained skin volume over the long term. Through this, it was confirmed that the mesenchymal stem cell spheroids overcome the limitations of existing cell-based therapies and exhibit more sustained effects as a dermal filler.

### [Disclosure]

### [Technical Problem]

An object of the present disclosure is to provide a pharmaceutical composition for treating skin wounds or scars, comprising a therapeutically effective amount of mesenchymal stem cell spheroids, which are applied topically to a target site or administered subcutaneously or intradermally.

Another object of the present disclosure is to provide a pharmaceutical composition for treating skin wounds or scars, comprising mesenchymal stem cell spheroids and a pharmaceutically acceptable carrier.

Still another object of the present disclosure is to provide a filler composition for skin regeneration, comprising mesenchymal stem cell spheroids.

Still another object of the present disclosure is to provide the use of mesenchymal stem cell spheroids according to the present disclosure for the manufacture of a medicament for treating skin wounds or scars, which is applied topically to a target site or administered subcutaneously or intradermally.

Still another object of the present disclosure is to provide mesenchymal stem cell spheroids for use in treating skin wounds or scars by topical application to a target site or by subcutaneous or intradermal injection.

Still another object of the present disclosure is to provide a method for treating skin wounds or scars by topical application, or by subcutaneous or intradermal injection, of a therapeutically effective amount of mesenchymal stem cell spheroids to a target site of a subject in need of treatment.

### [Technical Solution]

Unless otherwise defined, all terms (including technical and scientific terms) used herein have the same meaning as commonly understood by one of ordinary skill in the relevant art. It is also understood that terms defined in commonly used dictionaries should be interpreted as having a meaning consistent with their meaning in the relevant technical context and will not be interpreted in an idealized or overly formal sense unless explicitly so defined herein.

The terminology used herein is for the purpose of describing particular embodiments only and is not intended to be limiting. As used herein, the singular form is intended to include the plural form as well, unless the context clearly indicates otherwise. Furthermore, to the extent that the terms "including," "include," "having," "have", "with," or variations thereof are used in either the description and/or claims, such terms are intended to be inclusive in a manner similar to the term "comprising."

Unless otherwise indicated herein, references to ranges of values are intended to serve merely as a shorthand method of referring individually to each individual value falling within the range, and each individual value is incorporated into the specification as if it were individually recited herein. The use of any examples or representative language (e.g., "such as") provided herein is intended merely to better illuminate the invention and does not pose a limitation on the scope of the invention unless otherwise stated. No language in the specification should be construed as indicating any non-claimed element as essential to the practice of the invention.

The inventors of the present disclosure confirmed that mesenchymal stem cell spheroids, prepared by 3D culturing adipose tissue-derived mesenchymal stem cells, exhibit excellent expression level and production ability of collagen type I (ColI), fibronectin (FN1), hyaluronic acid (HA), glycosaminoglycan (GAG), tenascin (TNC), and proteoglycan-4 (PRG4). Accordingly, it was confirmed that the mesenchymal stem cell spheroids of the present disclosure are excellent for skin regeneration and the treatment of skin wounds and scars, and, in particular, have the optimal dose and administration method for exhibiting therapeutic efficacy.

In one general aspect, the present disclosure relates to a pharmaceutical composition for treating skin wounds or scars, comprising a therapeutically effective amount of mesenchymal stem cell spheroids, which are applied topically to a target site or administered subcutaneously or intradermally.

### Mesenchymal Stem Cell Spheroid

The mesenchymal stem cell spheroids of the present disclosure may be produced by culturing mesenchymal stem cells (hMSCs) in two dimensions and using three-dimensional culture techniques.

In an embodiment, the mesenchymal stem cell spheroids of the present disclosure (also referred to herein as MiBs) may be produced by isolating mesenchymal stem cells (hMSCs) from human adipose tissue, followed by culturing the isolated stem cells in two dimensions, and then applying three-dimensional culture techniques.

The term "two-dimensional (2D) culture" refers to culturing cells as a monolayer in any type of culture vessel, such as a cell culture flask or flat petri dish.

The "three-dimensional (3D) culture" refers to a cell culture method in which cells interact with their surroundings in all three spatial dimensions, allowing them to grow and organize in all directions.

The above "mesenchymal stem cell spheroid (MiB)" refers to a unit spheroid of various sizes that is formed through cellular self-assembly.

In the present disclosure, the mesenchymal stem cell spheroid refers to a three-dimensional cell aggregate of mesenchymal stem cells produced by culturing mesenchymal stem cells, wherein the cells are aggregated, rather than dissociated, to form a three-dimensional cell tissue. More preferably, it is a three-dimensional cell aggregate of adipose-derived mesenchymal stem cells.

The mesenchymal stem cell spheroid may be obtained by culturing adult stem cells. The adult stem cells may be commercially available stem cells or those isolated from living tissue.

The mesenchymal stem cell spheroid may effectively induce skin regeneration and healing of skin wounds and scars. Growth factor expression and collagen production associated with skin regeneration and skin wound and scar healing through mesenchymal stem cell spheroid treatment are significantly higher than those of single mesenchymal stem cell (MSC) treatment.

In the present disclosure, the diameter of the mesenchymal stem cell spheroid may range from 10 to 800 µm, preferably from 20 to 600 µm, more preferably from 30 to 400 µm, even more preferably from 60 to 300 µm, and even more preferably from 80 to 200 µm.

In the present disclosure, the number of mesenchymal stem cells contained in one mesenchymal stem cell spheroid may range from 200 to 800 cells, preferably 250 to 750 cells, more preferably 300 to 700 cells, and even more preferably 400 to 600 cells.

In the present disclosure, the wet weight of the mesenchymal stem cell spheroid may be 1 to 6 µg, preferably 2 to 5 µg, more preferably 2.5 to 4 µg, and even more preferably 3.19±0.5 µg. The wet weight of the mesenchymal stem cell spheroid refers to the wet weight of a single mesenchymal stem cell spheroid.

In the present disclosure, the dry weight of the mesenchymal stem cell spheroid may be 250 to 500 ng, preferably 280 to 450 ng, more preferably 300 to 400 ng, and even more preferably 303 to 370 ng. The dry weight of the mesenchymal stem cell spheroid refers to the dry weight of a single mesenchymal stem cell spheroid.

In the present disclosure, the mesenchymal stem cells may be any one or more selected from the group consisting of adipose-derived mesenchymal stem cells, bone marrow-derived mesenchymal stem cells, umbilical cord blood-derived mesenchymal stem cells, umbilical cord-derived mesenchymal stem cells, and peripheral blood-derived mesenchymal stem cells. Preferably, the mesenchymal stem cells may be adipose-derived mesenchymal stem cells, and more preferably, human adipose-derived mesenchymal stem cells.

In the present disclosure, the mesenchymal stem cell spheroid may comprise mesenchymal stem cells (MSCs) and an extracellular matrix (ECM). Specifically, the extracellular matrix may comprise collagen, fibronectin, hyaluronic acid (HA), elastin (EL), or glycosaminoglycan (GAG).

In addition, the collagen may preferably be type 1 collagen.

More specifically, the mesenchymal stem cell spheroids according to the present disclosure may exhibit higher expression of collagen, fibronectin, hyaluronic acid (HA), elastin (EL), or glycosaminoglycan (GAG), compared to single mesenchymal stem cells or dermal fibroblasts.

In the present disclosure, the mesenchymal stem cell spheroids may exhibit increased expression of any one or more factors selected from the group consisting of fibroblast growth factor 2 (FGF2), platelet-derived growth factor subunit A (PDGFA), vascular endothelial growth factor A (VEGFA), hepatocyte growth factor (HGF), and insulin-like growth factor 1 (IGF-1).

Preferably, the mesenchymal stem cell spheroid may have increased expression of vascular endothelial growth factor A (VEGFA), hepatocyte growth factor (HGF), or both.

In addition, preferably, the mesenchymal stem cell spheroids may maintain the expression of fibroblast growth factor 2 (FGF2) or platelet-derived growth factor subunit A (PDGFA).

More specifically, the mesenchymal stem cell spheroids according to the present disclosure may exhibit a higher expression of at least one factor selected from the group consisting of vascular endothelial growth factor A (VEGFA), hepatocyte growth factor (HGF), and insulin-like growth factor 1 (IGF-1) compared to mesenchymal stem cells or dermal fibroblasts.

Further, the mesenchymal stem cell spheroid according to the present disclosure may express fibroblast growth factor 2 (FGF2) or platelet-derived growth factor subunit A (PDGFA) at a level equivalent to that of mesenchymal stem cells or dermal fibroblasts.

In the present disclosure, the mesenchymal stem cell spheroid is positive for at least one marker selected from the group consisting of CD29, CD44, CD73, CD90, and CD105, and negative for at least one marker selected from the group consisting of CD31, CD45, CD79a, CD117, and HLA-DR.

The mesenchymal stem cell spheroid may have cells that are positive for expression of any one or more markers selected from the group consisting of CD29, CD44, CD73, CD90, and CD105 by at least 85%, 86%, 87%, 88%, 89%, 90%, 91%, 92%, 93%, 94%, 95%, 96%, 97%, 98%, or 99% or more. Further, the mesenchymal stem cell spheroid may have cells that are negative for expression of any one or more markers selected from the group consisting of CD31, CD45, CD79a, CD117, and HLA-DR by at least 10%, 9%, 8%, 7%, 6%, 5%, 3%, 2%, or less.

More specifically, the mesenchymal stem cell spheroid may have cells that are positive for expression of CD29, CD44, CD73, CD90, and CD105 by at least 85%, 86%, 87%, 88%, 89%, 90%, 91%, 92%, 93%, 94%, 95%, 96%, 97%, 98%, or 99% or more. Furthermore, the mesenchymal stem cell spheroid may have cells that are negative for expression of CD31, CD45, CD79a, CD117, and HLA-DR by at least 10%, 9%, 8%, 7%, 6%, 5%, 3%, 2%, or less.

Furthermore, the mesenchymal stem cell spheroid may have cells that are positive for expression of CD29, CD44, CD73, CD90, and CD105 by at least 95%, 96%, 97%, 98%, or 99% or more. In addition, the mesenchymal stem cell spheroid may have cells that are negative for expression of CD31, CD45, CD79a, CD117, and HLA-DR by at least 2%, 1.5%, 1% or less.

The mesenchymal stem cell spheroids of the present disclosure, being derived from adipose tissue, are positive for expression of the markers CD29, CD44, CD73, CD90, and CD105, and are negative for expression of the markers CD31, CD45, CD79a, CD117, and HLA-DR.

The mesenchymal stem cell spheroid of the present disclosure may express any one or more proteins selected from the group consisting of Tenascin (TNC), Fibronectin (FN1), Proteoglycan-4 (PRG4), COL5A3 (Collagen Type V Alpha 3 Chain), COL7A1 (Collagen Type VII Alpha 1 Chain), EVPL (Envoplakin), EFEMP2 (Fibulin-4), MFAP2 (Microfibril Associated Protein 2), FBLN2 (Fibulin-2), HSPG2, FBLN1 (Fibulin-1), and COL6A3 (Collagen alpha-3(VI) chain).

Specifically, the mesenchymal stem cell spheroids of the present disclosure exhibit significantly higher expression levels of tenascin (TNC) and fibronectin (FN1) compared to single mesenchymal stem cells.

Furthermore, the mesenchymal stem cell spheroids of the present disclosure may specifically express proteoglycan-4 (PRG4) and may newly express proteins, such as COL5A3 (Collagen Type V Alpha 3 Chain), COL7A1 (Collagen Type VII Alpha 1 Chain), EVPL (Envoplakin), EFEMP2 (Fibulin-4), and MFAP2 (Microfibril Associated Protein 2), compared to single cells.

The increase in expression level described above may indicate, for example, an increase in the level of any of the proteins described above or their genes by at least 5%, 6%, 7%, 8%, 9%, 10%, 20%, 30%, 40%, 50%, 60%, 70%, 80%, 90%, 100%, 150%, 200%, 300%, 400%, 500%, 600%, 700%, 800%, 900%, 1000%, or more compared to typical mesenchymal stem cells.

In some embodiments, the mesenchymal stem cell spheroids used in the treatment are also referred to as TRTP-101.

### Composition for Treating Wounds or Scars Comprising Mesenchymal Stem Cell Spheroids

The present disclosure provides a pharmaceutical composition or cell therapy composition for treating skin wounds or scars, comprising a therapeutically effective amount of mesenchymal stem cell spheroids.

The pharmaceutical composition may be applied topically to, or injected subcutaneously or intradermally into an area requiring skin regeneration, specifically, recovery of the entire skin layer up to the dermis or subcutaneous layer, thereby treating skin wounds or scars.

In the present disclosure, the pharmaceutical composition may be formulated into various dosage forms such as injection, infusion, spray, liquid, suspension, external use or patch, and preferably may be formulated into injection, infusion, suspension, or external use.

The term "treatment" refers to any action that improves or beneficially alters the symptoms of the disease by administering mesenchymal stem cell spheroids according to the present disclosure. For example, it may provide skin regeneration or wound or scar healing in a shorter period of time compared to natural healing. The treatment may include the improvement and/or relief of wounds or scars. Improvement of wounds or scars refers to the alleviation of wound or scar severity. Further, the treatment may encompass the treatment of both wounds and/or wound-related diseases. The treatment may refer to the healing and/or regeneration of damaged tissue resulting from the wound. The wound treatment may refer to skin regeneration, specifically, the restoration of the dermis within the skin. Furthermore, the treatment may maintain the original composition of the damaged tissue. In addition, the treatment may promote the healing and/or regeneration of damaged tissue while minimizing complications and/or scarring of wound-related diseases.

In addition, the present disclosure provides a pharmaceutical composition for skin regeneration, skin wound or scar treatment, comprising mesenchymal stem cell spheroids and a pharmaceutically acceptable carrier.

The pharmaceutical composition for skin regeneration, and skin wound and scar treatment may comprise a conventional, pharmaceutically acceptable, inert carrier or diluent. Pharmaceutically acceptable carriers and diluents that may be included in the pharmaceutical composition of the present disclosure include, but are not limited to, excipients such as starch, sugar, and mannitol; fillers and extenders such as calcium phosphate; cellulose derivatives such as carboxymethylcellulose and hydroxypropylcellulose; binders such as gelatin, alginates, and polyvinyl pyrrolidone; lubricants such as talc, calcium stearate, hydrogenated castor oil, and polyethylene glycol; disintegrants such as povidone and crospovidone; and surfactants such as polysorbate, cetyl alcohol, and glycerol. The pharmaceutically acceptable carrier and diluent may be biologically and physiologically compatible with the recipient. Examples of diluents include, but are not limited to, saline, aqueous buffers, solvents, suspending agents, and/or dispersion media. In addition, for example, injectable formulations may further include preservatives, analgesics, solubilizers, suspending agents, or stabilizers, while topical formulations may further include bases, excipients, lubricants, suspending agents, or preservatives. In addition, in an embodiment, the suspending agent that is additionally contained includes, but is not limited to, DMEM (Dulbecco's Modified Eagle's Medium), MEM (Minimal Essential Medium), BME (Basal Medium Eagle), RPMI 1640, F-10, F-12, DMEM/F12, α-MEM (α-Minimal Essential Medium), G-MEM (Glasgow's Minimal Essential Medium), IMDM (Iscove's Modified Dulbecco's Medium), MacCoy's 5A medium, AmnioMax complete medium, AminoMax ± complete medium, EBM (Endothelial Basal Medium) medium, Chang's Medium, MesenCult-XF, DMEM/HG (Dulbecco's Modified Eagle's Medium high glucose) medium, or MCDB+DMEM/LG (MCDB+Dulbecco's Modified Eagle's Medium low glucose). The composition of the present disclosure may be used unfrozen or frozen for later use. If freezing is required, standard cryopreservatives (e.g., DMSO, glycerol, Epilife^{®} Cell Freezing Medium (Cascade Biologics)) may be added to the cell population prior to freezing. Furthermore, in an embodiment, the topical formulation may be a cream, gel, ointment, skin emulsifier, skin suspension, transdermal patch, drug-containing bandage, lotion, or a combination thereof. The topical formulation may be prepared by appropriately blending ingredients commonly used in topical formulations such as pharmaceuticals, such as aqueous ingredients, oily ingredients, powdered ingredients, alcohols, moisturizers, thickeners, UV absorbers, whitening agents, preservatives, antioxidants, surfactants, fragrances, colorants, and various skin nutrients, as needed. The topical formulation may be prepared by appropriately blending metal sequestrants such as disodium edetate, trisodium edetate, sodium citrate, sodium polyphosphate, sodium metaphosphate, and gluconic acid; caffeine, tannin, verapamil, licorice extract, glabridine, hot water extract of the fruit of calines, various herbal medicines, drugs such as tocopherol acetate, glycyrrhetinic acid, tranexamic acid and derivatives or salts thereof; vitamin C, magnesium ascorbyl phosphate, ascorbyl glucoside, arbutin, kojic acid, sugars such as glucose, fructose, and trehalose; and the like.

The blending ratio of the pharmaceutical composition of an embodiment of the present disclosure may be appropriately selected depending on the type, amount, form, etc., of the additional ingredients described above.

### Administration Method and Dose of Mesenchymal Stem Cell Spheroids for Treating Skin Wounds or Scars

The present disclosure provides a method for treating skin wounds or scars by topical application, or by subcutaneous or intradermal injection, of a therapeutically effective amount of mesenchymal stem cell spheroids to a target site of a subject in need of treatment.

The mesenchymal stem cell spheroids of the present disclosure may be administered in a therapeutically effective amount to a subject requiring skin wound or scar treatment, thereby promoting skin regeneration and treating skin wounds or scars.

The mesenchymal stem cell spheroids of the present disclosure or a pharmaceutical composition comprising the same may be administered directly or indirectly using administration methods commonly used in the art. In an embodiment of the present disclosure, the mesenchymal stem cell spheroids may be administered subcutaneously, transdermally (by application), or intradermally. Furthermore, the administration may mean direct administration to a target site requiring skin regeneration through collagen production for the treatment of skin wounds or scars.

In the present disclosure, the target site may include a damaged or depressed area in a cell layer constituting the skin, such as the epidermis, dermis, or subcutaneous layer. Preferably, the target site may include a damaged or depressed area in the dermis, or a damaged or depressed area in the subcutaneous layer. Specifically, damage or depression of the dermis refers to damage or depression of the epidermis layer, which constitutes the skin, to the dermis layer. Damage or depression of the subcutaneous layer refers to damage or depression of the entire skin layer, including the epidermis, dermis, and subcutaneous layers.

The application described above refers to any method of contacting the target site with the mesenchymal stem cell spheroid or a pharmaceutical composition containing the same using an appropriate method, thereby allowing the composition to be absorbed into the skin.

In the present disclosure, the mesenchymal stem cell spheroids may be administered in a single dose or in multiple doses.

In the present disclosure, the mesenchymal stem cell spheroid may be administered once, twice, three times, four times, five times, or more times. Preferably, the mesenchymal stem cell spheroids may be administered once, twice, or three times. More preferably, a single administration is preferred. The mesenchymal stem cell spheroids of the present disclosure are effective in skin regeneration and treating skin wounds or scars even with a single administration.

The "therapeutically effective amount" refers to an amount sufficient to exhibit a therapeutic effect when administered to a subject in need of skin regeneration or treatment of skin wounds or scars.

In terms of the administration dose for administration to a subject, the mesenchymal stem cell spheroids may be contained in a unit dosage of 1 to 2 × 10⁵, for example, the lower limit thereof may be 1 or more, or 2 or more, and the upper limit thereof may be 2 × 10⁵ or less, 1.8 × 10⁵ or less, 1.6 × 10⁵ or less, 1.4 × 10⁵ or less, 1.2 × 10⁵ or less, 1 × 10⁵ or less, 9.5 × 10⁴ or less, 9 × 10⁴ or less, 8.5 × 10⁴ or less, 8 × 10⁴ or less, 7.5 × 10⁴ or less, 7 × 10⁴ or less, 6.5 × 10⁴ or less, 6 × 10⁴ or less, 5.5 × 10⁴ or less, 5 × 10⁴ or less, 4.5 × 10⁴ or less, 4 × 10⁴ or less, 3.5 × 10⁴ or less, 3 × 10⁴ or less, 2.5 × 10⁴ or less, 2 × 10⁴ or less, 1.5 × 10⁴ or less, 1 × 10⁴ or less, 9.5 × 10³ or less, 9 × 10³ or less, 8.5 × 10³ or less, 8 × 10³ or less, 7.5 × 10³ or less, 7 × 10³ or less, 6.5 × 10³ or less, 6 × 10³ or less, 5.5 × 10³ or less, 5 × 10³ or less, 4.5 × 10³ or less, 4 × 10³ or less, 3.5 × 10³ or less, 3 × 10³ or less, 2.5 × 10³ or less, or 2 × 10³ or less. Here, the number of mesenchymal stem cells contained in one mesenchymal stem cell spheroid may range from 200 to 800 cells, preferably 250 to 750 cells, more preferably 300 to 700 cells, and even more preferably 400 to 600 cells of mesenchymal stem cells.

Specifically, the number of mesenchymal stem cells contained in a unit dosage may range from 1 × 10² cells to 8 × 10⁷ cells, for example, the lower limit thereof may be 1.0 × 10² cells or more, 1.2 × 10² cells or more, 1.4 × 10² cells or more, 1.6 × 10² cells or more, 1.8 × 10² cells or more, 2.0 × 10² cells or more, 2.2 × 10² cells or more, 2.4 × 10² cells or more, 2.6 × 10² cells or more, 2.8 × 10² cells or more, 3.0 × 10² cells or more, 3.2 × 10² cells or more, 3.4 × 10² cells or more, 3.6 × 10² cells or more, 3.8 × 10² cells or more, 4.0 × 10² cells or more, 4.2 × 10² cells or more, 4.4 × 10² cells or more, 4.6 × 10² cells or more, 4.8 × 10² cells or more, 5.0 × 10² cells or more, 6.0 × 10² cells or more, 8.0 × 10² cells or more, 1.0 × 10³ cells or more, 1.2 × 10³ cells or more, 1.4 × 10³ cells or more, 1.6 × 10³ cells or more, 1.8 × 10³ cells or more, 2.0 × 10³ cells or more, 2.2 × 10³ cells or more, 2.4 × 10³ cells or more, 2.6 × 10³ cells or more, 2.8 × 10³ cells or more, 3.0 × 10³ cells or more, 3.2 × 10³ cells or more, 3.4 × 10³ cells or more, 3.6 × 10³ cells or more, or 3.8 × 10³ cells or more, and the upper limit thereof may be 8 × 10⁷ cells or less, 8.6 × 10⁷ cells or less, 8.4 × 10⁷ cells or less, 8.2 × 10⁷ cells or less, 8 × 10⁷ cells or less, 7.8 × 10⁷ cells or less, 7.6 × 10⁷ cells or less, 7.4 × 10⁷ cells or less, 7.2 × 10⁷ cells or less, 7 × 10⁷ cells or less, 6.8 × 10⁷ cells or less, 6.6 × 10⁷ cells or less, 6.4 × 10⁷ cells or less, 6.2 × 10⁷ cells or less, 6 × 10⁷ cells or less, 5.5 × 10⁷ cells or less, 5 × 10⁷ cells or less, 4.5 × 10⁷ cells or less, 4 × 10⁷ cells or less, 3.5 × 10⁷ cells or less, 3 × 10⁷ cells or less, 2.5 × 10⁷ cells or less, 2 × 10⁷ cells or less, 1.5 × 10⁷ cells or less, 1 × 10⁷ cells or less, 9 × 10⁶ cells or less, 8 × 10⁶ cells or less, 7 × 10⁶ cells or less, 6 × 10⁶ cells or less, 5 × 10⁶ cells or less, 4 × 10⁶ cells or less, 3 × 10⁶ cells or less, 2.0 × 10⁶ cells, or 1.5 × 10⁶ cells or less.

In the present disclosure, the unit dosage may be 50 uL, 60 uL, 70 uL, 80 uL, 90 uL, 100 uL, 125 uL, 150 uL, 175 uL, 200 uL, 225 uL, 250 uL, 275 uL, 300 uL, 325 uL, 350 uL, 375 uL, 400 uL, 425 uL, 450 uL, 475 uL, or 500 uL.

In an embodiment, the mesenchymal stem cell spheroids may be contained in an amount of 1 to 2 × 10⁵ based on the unit dosage per volume of the target site, for example, the lower limit thereof may be 1 or more, or 2 or more, and the upper limit thereof may be 2 × 10⁵ or less, 1.8 × 10⁵ or less, 1.6 × 10⁵ or less, 1.4 × 10⁵ or less, 1.2 × 10⁵ or less, 1 × 10⁵ or less, 9.5 × 10⁴ or less, 9 × 10⁴ or less, 8.5 × 10⁴ or less, 8 × 10⁴ or less, 7.5 × 10⁴ or less, 7 × 10⁴ or less, 6.5 × 10⁴ or less, 6 × 10⁴ or less, 5.5 × 10⁴ or less, 5 × 10⁴ or less, 4.5 × 10⁴ or less, 4 × 10⁴ or less, 3.5 × 10⁴ or less, 3 × 10⁴ or less, 2.5 × 10⁴ or less, 2 × 10⁴ or less, 1.5 × 10⁴ or less, 1 × 10⁴ or less, 9.5 × 10³ or less, 9 × 10³ or less, 8.5 × 10³ or less, 8 × 10³ or less, 7.5 × 10³ or less, 7 × 10³ or less, 6.5 × 10³ or less, 6 × 10³ or less, 5.5 × 10³ or less, 5 × 10³ or less, 4.5 × 10³ or less, 4 × 10³ or less, 3.5 × 10³ or less, 3 × 10³ or less, 2.5 × 10³ or less, or 2 × 10³ or less.

Here, the target site may have a volume of 0.2 to 500 mm³, preferably 0.3 to 500 mm³ or 0.4 to 500 mm³. The unit dosage may be administered at regular intervals depending on the area of the target site. Further, the number of mesenchymal stem cells contained in one of the above mesenchymal stem cell spheroids may range from 200 to 800 cells, preferably 250 to 750 cells, more preferably 300 to 700 cells, and even more preferably 400 to 600 cells of mesenchymal stem cells.

Specifically, the number of cells administered based on the unit dosage per volume of the target site may range from 1 × 10² cells to 8 × 10⁷ cells, for example, the lower limit thereof may be 1.0 × 10² cells or more, 1.2 × 10² cells or more, 1.4 × 10² cells or more, 1.6 × 10² cells or more, 1.8 × 10² cells or more, 2.0 × 10² cells or more, 2.2 × 10² cells or more, 2.4 × 10² cells or more, 2.6 × 10² cells or more, 2.8 × 10² cells or more, 3.0 × 10² cells or more, 3.2 × 10² cells or more, 3.4 × 10² cells or more, 3.6 × 10² cells or more, 3.8 × 10² cells or more, 4.0 × 10² cells or more, 4.2 × 10² cells or more, 4.4 × 10² cells or more, 4.6 × 10² cells or more, 4.8 × 10² cells or more, 5.0 × 10² cells or more, 6.0 × 10² cells or more, 8.0 × 10² cells or more, 1.0 × 10³ cells or more, 1.2 × 10³ cells or more, 1.4 × 10³ cells or more, 1.6 × 10³ cells or more, 1.8 × 10³ cells or more, 2.0 × 10³ cells or more, 2.2 × 10³ cells or more, 2.4 × 10³ cells or more, 2.6 × 10³ cells or more, 2.8 × 10³ cells or more, 3.0 × 10³ cells or more, 3.2 × 10³ cells or more, 3.4 × 10³ cells or more, 3.6 × 10³ cells or more, or 3.8 × 10³ cells or more, and the upper limit thereof may be 8 × 10⁷ cells or less, 8.6 × 10⁷ cells or less, 8.4 × 10⁷ cells or less, 8.2 × 10⁷ cells or less, 8 × 10⁷ cells or less, 7.8 × 10⁷ cells or less, 7.6 × 10⁷ cells or less, 7.4 × 10⁷ cells or less, 7.2 × 10⁷ cells or less, 7 × 10⁷ cells or less, 6.8 × 10⁷ cells or less, 6.6 × 10⁷ cells or less, 6.4 × 10⁷ cells or less, 6.2 × 10⁷ cells or less, 6 × 10⁷ cells or less, 5.5 × 10⁷ cells or less, 5 × 10⁷ cells or less, 4.5 × 10⁷ cells or less, 4 × 10⁷ cells or less, 3.5 × 10⁷ cells or less, 3 × 10⁷ cells or less, 2.5 × 10⁷ cells or less, 2 × 10⁷ cells or less, 1.5 × 10⁷ cells or less, 1 × 10⁷ cells or less, 9 × 10⁶ cells or less, 8 × 10⁶ cells or less, 7 × 10⁶ cells or less, 6 × 10⁶ cells or less, 5 × 10⁶ cells or less, 4 × 10⁶ cells or less, 3 × 10⁶ cells or less, 2.0 × 10⁶ cells, or 1.5 × 10⁶ cells or less.

Here, the target site may have a volume of 0.2 to 500 mm³, preferably 0.3 to 500 mm³ or 0.4 to 500 mm³. The unit dosage may be administered at regular intervals depending on the area of the target site.

In the present disclosure, after administration of the composition, the target site may be treated or recovered, thereby enabling skin regeneration and the treatment of wounds and scars. The composition of the present disclosure may exhibit skin regeneration and wound and scar treatment efficacy for a long period of time from the time of administration after a single administration.

In the present disclosure, skin regeneration may refer to the improvement of changes in the skin, such as deep or shallow wrinkles, caused by the passage of time, light, cosmetics, soap, pollution, dust, dryness, tobacco, lifestyle habits, etc.

In the present disclosure, the scar may be any one or more selected from the group consisting of bedsore scars, burn scars, hair loss scars, linear scleroderma scars, wound scars, diabetic foot ulcer scars, atrophic scars, and stretch marks. Furthermore, examples of the scar may include scars in which the epidermis; dermis; subcutaneous; epidermis and dermis; dermis and subcutaneous; or epidermis, dermis and subcutaneous layers of the skin are damaged. Preferably, the scar may be a full-thickness scar with damage to the dermis, or a full-thickness scar with damage to the epidermis, dermis, and subcutaneous layer.

In the present disclosure, the wound refers to an injury to the human body resulting from tissue being cut, torn, broken, burned, or traumatized, or from a disorder or disease that causes such damage. The wound may be an open wound with an open surface or a closed wound with no open surface. An example of the wound may be an open wound on the skin. Examples of the wound may include wounds in which the epidermis; the dermis; the epidermis and the dermis; or the epidermis, dermis, and subcutaneous fat layers are damaged. Preferably, the wound may be a full-thickness wound with damage to the dermis, or a full-thickness wound with damage to the epidermis, dermis, and subcutaneous layer.

Further, examples of the wound may include cuts, incisions (e.g., surgical incisions), wounds, abrasions, contusions, penetrating wounds, incised wounds, avulsion injuries, lacerations, fractures, burns, and amputations.

More specifically, the mesenchymal stem cell spheroid according to the present disclosure may be directly administered once to a scar or wound site as a single administration dose, and the administration dose is as described above.

The present disclosure provides a filler composition for skin regeneration comprising mesenchymal stem cell spheroids.

The present disclosure provides the use of mesenchymal stem cell spheroids for the preparation of a medicament for treating skin wounds or scars, which is applied topically to a target site or administered subcutaneously or intradermally.

The present disclosure provides mesenchymal stem cell spheroids for use in the treatment of skin wounds or scars by topical application to a target site or by subcutaneous or intradermal injection.

The present disclosure provides a method for treating skin wounds or scars by topical application, or by subcutaneous or intradermal injection, of a therapeutically effective amount of mesenchymal stem cell spheroids to a target site of a subject in need of treatment.

### [Advantageous Effects]

The composition comprising the mesenchymal stem cell spheroids of the present disclosure may promote wound healing by directly administering a therapeutically effective amount to a wound, scar, or skin depression requiring dermal or subcutaneous recovery and skin regeneration, and may promote skin regeneration by inducing dermal and subcutaneous recovery within the skin, thereby enhancing scar healing/recovery and maintenance of skin volume.

### [Description of Drawings]

FIG. 1 shows the microscopic morphology of mesenchymal stem cells isolated from adipose tissue.
FIG. 2 shows the cell growth rate of mesenchymal stem cells isolated from adipose tissue.
FIG. 3 shows the purity of mesenchymal stem cells confirmed by flow cytometry, indicating negative and positive markers.
FIG. 4 shows the staining results verifying the differentiation potential of adipocytes, osteocytes, and chondrocytes, which are characteristics of mesenchymal stem cells.
FIG. 5 shows the RT-PCR results verifying the differentiation potential of mesenchymal stem cells into adipocytes, osteocytes, and chondrocytes, which are characteristics of mesenchymal stem cells.
FIG. 6 shows microscopic images of mesenchymal stem cell spheroids, spherical cell structures formed by self-assembly of mesenchymal stem cells, at different culture days.
FIG. 7 shows the size distribution of mesenchymal stem cell spheroids by donor and passage.
FIG. 8 shows the cell viability of mesenchymal stem cell spheroids by donor and passage.
FIG. 9 shows the DNA content of mesenchymal stem cell spheroids by incubation period.
FIG. 10 shows electron microscopic images of mesenchymal stem cell spheroids.
FIG. 11 shows the expression of mesenchymal stem cell markers in cells constituting the mesenchymal stem cell spheroids, as confirmed by flow cytometry.
FIG. 12 shows microscopic images of mesenchymal stem cell spheroids stained with immunofluorescence for mesenchymal stem cell markers from each donor.
FIG. 13 shows the results of quantitative RT-PCR analysis of extracellular matrix expression level in mesenchymal stem cell spheroids.
FIG. 14 shows a graph of extracellular matrix expression in mesenchymal stem cell spheroids measured by enzyme-linked immunosorbent assay.
FIG. 15 shows fluorescent immunostaining images of collagen expression in mesenchymal stem cell spheroids.
FIG. 16 shows histological analysis images of collagen expression in mesenchymal stem cell spheroids.
FIG. 17 shows the results of real-time RT-PCR analysis of growth factor expression in mesenchymal stem cell spheroids.
FIG. 18 shows the results of collagen area analysis in the test and control groups of wound-induced animal models administered with mesenchymal stem cell spheroids, wherein FIG. 18A shows the results of intradermal injection of mesenchymal stem cell spheroids, and FIG. 18B shows the results of topical application of mesenchymal stem cell spheroids.
FIG. 19 shows the results of a comprehensive scoring of safety and efficacy indicators in the test and control groups of wound-induced animal models administered intradermally with mesenchymal stem cell spheroids.
FIG. 20 shows the results of analyzing the wound area ratio in the test and control groups of wound-induced animal models administered subcutaneously with mesenchymal stem cell spheroids.
FIG. 21 shows the results of a histopathological analysis in the test and control groups of wound-induced animal models administered subcutaneously with mesenchymal stem cell spheroids.
FIG. 22 shows the results of the collagen and type 1 collagen areas in the test and control groups of normal nude mice administered with mesenchymal stem cell spheroids.
FIG. 23 shows the results of dermal thickness analysis in the test and control groups of normal nude mice administered with mesenchymal stem cell spheroids.
FIG. 24 shows the results of an in vitro comparative analysis of the production ability of type 1 collagen and FN in mesenchymal stem cell spheroids (TRTP-101), HDF, and MSC single cells (SCs).
FIG. 25 shows the results of an in vitro comparative analysis of the growth factor protein expression levels in mesenchymal stem cell spheroids (TRTP-101), HDF, and MSC single cells (SCs).

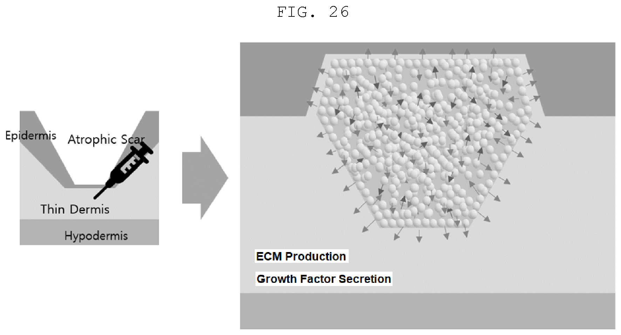

FIG. 26 is a schematic diagram illustrating the application and recovery of mesenchymal stem cell spheroids of the present disclosure to skin wounds or scars.
FIG. 27 shows Antera 3D^{®} images of atrophic scar sites before and after administration (1 week, 4 weeks, and 8 weeks) of the mesenchymal stem cell spheroids of the present disclosure.
FIG. 28 shows the volume of atrophic scars measured before and after administration (1 week, 4 weeks, and 8 weeks) of the mesenchymal stem cell spheroids of the present disclosure.
FIG. 29 shows the inter-experimenter variation in dry weight measurements of the mesenchymal stem cell spheroids (MiBs) of the present disclosure.
FIG. 30 shows a linear trend line for the dry weight of the mesenchymal stem cell spheroids (MiBs) of the present disclosure.
FIG. 31 shows the results of a total protein count analysis of mesenchymal stem cell spheroids of the present disclosure and single cells.
FIG. 32 shows the results of a principal component analysis of mesenchymal stem cell spheroids of the present disclosure and single cells.
FIG. 33 shows the results of a heatmap analysis of mesenchymal stem cell spheroids of the present disclosure and single cells.
FIG. 34 shows the results of the protein increase/decrease distributions of mesenchymal stem cell spheroids of the present disclosure and single cells.

### [Best Mode]

To achieve the above objectives, the present disclosure provides a method for producing mesenchymal stem cell spheroids using mesenchymal stem cells isolated from adipose tissue.

Hereinafter, embodiments of the present disclosure will be described in detail with reference to the accompanying drawings so that those skilled in the art can easily implement the present disclosure. However, the present disclosure may be embodied in various forms and is not limited to the embodiments described herein.

The following Examples will further illustrate the present disclosure. However, these Examples are provided for illustrative purposes only and are not intended to limit the scope of the present disclosure.

### Example 1. Manufacturing and Characterization of Adipose-Derived Mesenchymal Stem Cell Spheroids (MiBs)

### Example 1-1. Isolation of Mesenchymal Stem Cells (hMSCs) from Adipose Tissue

Human adipose-derived mesenchymal stem cells (hMSCs), which are relatively easy to isolate and have a high growth rate, were used as the cell source for producing the mesenchymal stem cell spheroids (Microblocks, MiBs) of the present disclosure. The isolation of hMSCs was conducted with approval from the Institutional Review Board of Seoul National University Dental Hospital.

The prepared human adipose tissue was placed in Hanks^{®} Balanced Salt solution (HBSS) containing 0.1% collagenase I and shaken at 37°C for 30 minutes to isolate cells, followed by washing and filtering through a 100 µm nylon mesh. Then, the cells were cultured in a 5% CO₂ incubator at 37°C with nutrients supplied through Dulbecco's modified Eagle's medium (DMEM) containing 10% fetal bovine serum (FBS) and 1X antibiotic-antimycotic solution (AA).

### Example 1-2. Validation of Isolated Mesenchymal Stem Cells (hMSCs)

The hMSCs isolated in Example 1-1 exhibited characteristics such as adherence, multipotency, and expression of surface-specific antigens, and these characteristics were confirmed to verify that the cells isolated in Example 1-1 were hMSCs.

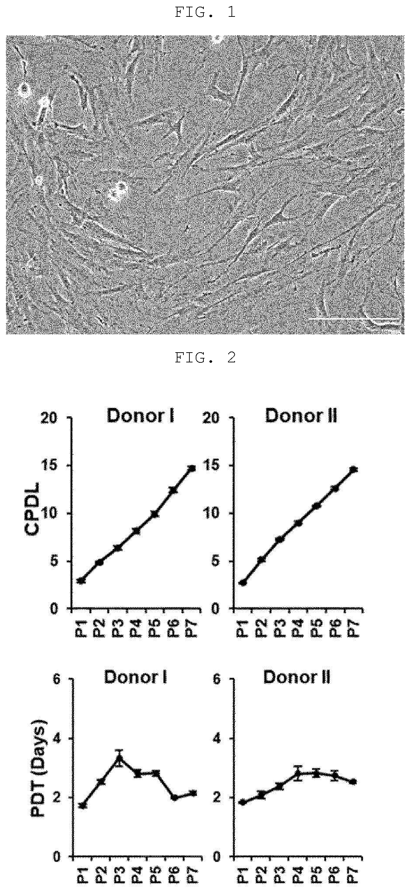

The morphological and distribution characteristics of the cells were confirmed. The cells were observed to grow in an attached state on plastic culture dishes with a homogeneous spindle-shaped morphology (FIG. 1), and were found to proliferate exponentially until the 7th passage, with division occurring approximately every 2-3 days (FIG. 2).

Furthermore, to confirm that hMSCs maintained their characteristics across passages, the expression of stem cell surface markers was determined using flow cytometry. Cultured MSCs were washed with PBS and treated with proteolytic enzymes such as trypsin and collagenase for 5 minutes at 37°C to harvest single cells. The cells were then incubated with fluorescently labeled antibodies against CD14, CD29, CD31, CD34, CD44, CD73, CD90, HLA-DR, CD45, CD79α, CD105, and CD117 for 1 hour at 4°C. Then, the cells were washed with Dulbecco's phosphate-buffered saline (DPBS) containing 2% FBS, and the expression level compared to the isotype mouse IgG control was analyzed by FACS.

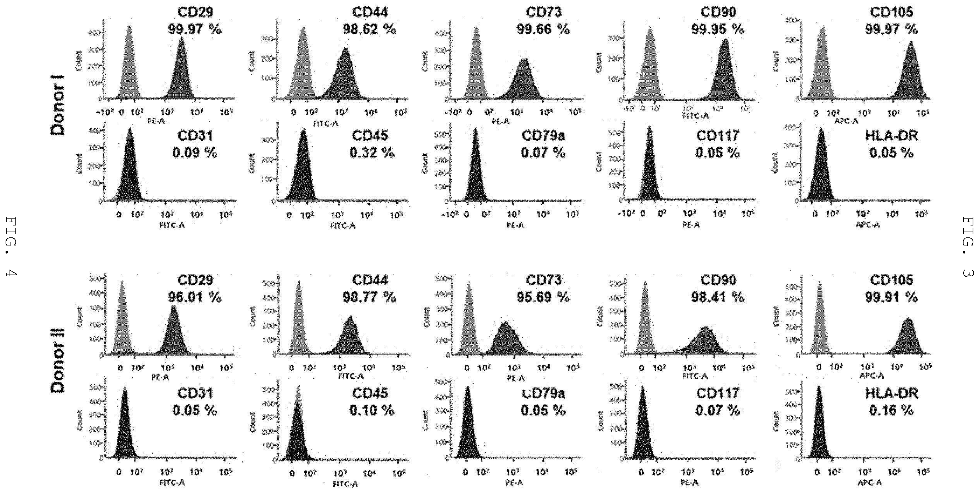

The analysis results showed that 95% or more of the cells were positive for the expression of positive markers CD29, CD44, CD73, CD90, and CD105, and less than 2% of the cells were positive for the expression of negative markers CD31, CD45, CD79a, CD117, and HLA-DR, confirming that the cells were MSCs separated with high purity (FIG. 3).

### Example 1-3. Adipogenic Differentiation Potential of Isolated Mesenchymal Stem Cells (hMSCs) Confirmed by Staining

To confirm adipogenic differentiation potential, 9.5 x 10⁴ cells were seeded in 6-well plates and cultured in high-glucose DMEM supplemented with 0.5 mM 3-isobutyl-1-methylxanthine (IBMX), 100 nM dexamethasone, 100 µM indomethacin (INDO), 10 µg/mL insulin, 10% FBS, and 1X AA. The cells were cultured for 14 days in a 5% CO₂ incubator at 37°C with media replacement twice a week. To confirm differentiation, the culture medium from the differentiation-completed plate was removed, washed once with DPBS, and fixed with 4% PFA at room temperature.

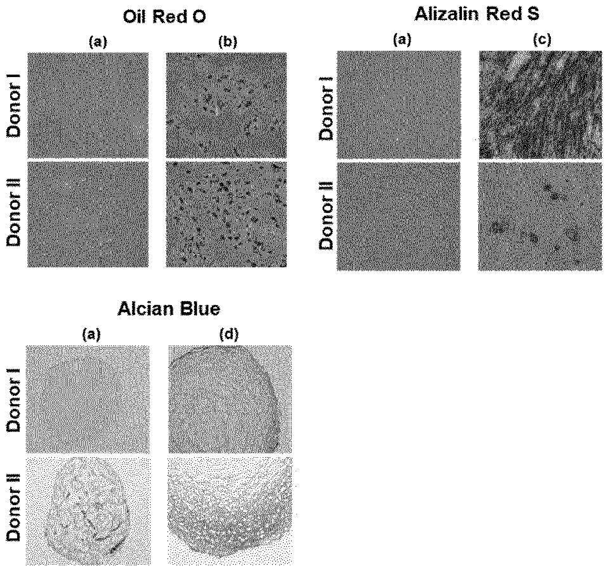

To visually confirm differentiation, 1 mL of a solution prepared by dissolving 0.2 g of oil red O powder in 40 mL of isopropanol and filtering through a 0.22 µm filter was added, incubated at room temperature for 15 minutes, and washed five times with deionized water, after which adipogenic differentiation was confirmed (FIG. 4).

### Example 1-4. Osteogenic Differentiation Potential of Isolated Mesenchymal Stem Cells (hMSCs) Confirmed by Staining

To confirm the osteogenic differentiation potential of hMSCs, 9.5 x 10⁴ cells were seeded in 6-well plates using the same method as in Example 1-3, and cultured in alpha minimum essential medium (α MEM) supplemented with 10 nM dexamethasone, 50 µg/mL ascorbic acid, 10 mM β-glycerolphosphate, 1 mM dibutyryl-cAMP, 10% FBS, and 1X AA. After 4 days, all culture medium was removed and replaced with medium without dibutyryl-cAMP twice a week. The cells were cultured in a 5% CO₂ incubator at 37°C for 14 days to induce osteogenic differentiation of hMSCs.

Subsequently, alizarin red S staining was performed to visually confirm osteogenic differentiation. After differentiation, the culture medium from the plate was removed and washed once with DPBS, and then 1 mL of 4% paraformaldehyde (PFA) was added and the cells were fixed at room temperature. The fixative was removed, and the cells were washed with DPBS, 2 mL of a 1% alizarin red S solution diluted in distilled water (DW) was added to each cell, and the cells were incubated at room temperature for 15 minutes. After washing five times with DW, 1 mL of DW was added, and osteogenic differentiation was confirmed under a microscope (Olympus) (FIG. 4).

### Example 1-5. Chondrogenic Differentiation Potential of Isolated Mesenchymal Stem Cells (hMSCs) Confirmed by Staining

In order to confirm chondrocyte differentiation potential, 9.5 x 10⁴ cells were seeded in 6-well plates using the same method as in Example 1-3, and cultured in α MEM containing 1X insulin transferrin selenium premix (ITS), 50 ng/mL ascorbic acid, 40 µg/mL L-proline, 100 nM dexamethasone, 10% FBS, and 1X AA supplemented with 10 ng/mL transforming growth factor (TGF-β3), in a 5% CO₂ incubator at 37°C for 21 days. After differentiation, the culture medium from the plate was removed, and the cells were fixed using 4% PFA at room temperature.

Once fixed, the cells were washed with DPBS, placed in OCT compound, and frozen in an ultra-low temperature freezer (-80°C) for one day. The frozen cells were sectioned to 8 µm thickness using a cryocut microtome and washed with deionized water. A 1% alcian blue solution prepared using alcian blue reagent was added to the sections, followed by staining for 30 minutes at room temperature and washing with a 0.1 N HCl solution and then with deionized water (DW), to confirm differentiation into chondrocytes (Chondrogenic differentiation) (FIG. 4).

### Example 1-6. Characteristics of Mesenchymal Stem Cells Verified by RT-PCR: Differentiation Potential into Adipocytes, Osteocytes, and Chondrocytes

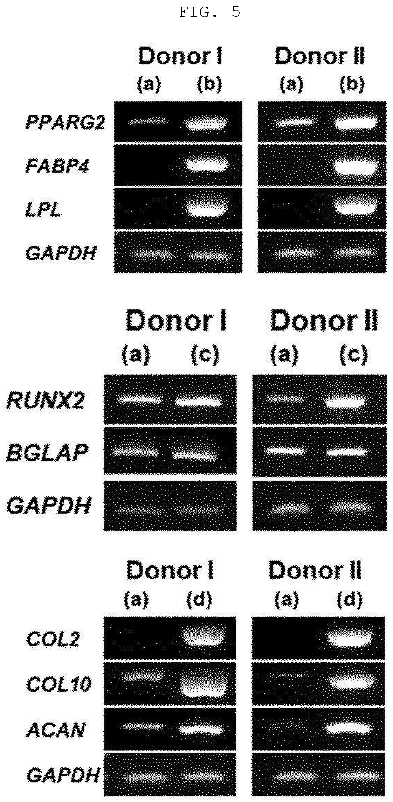

Total RNA was isolated from cells using TRI reagent with chloroform. cDNA was synthesized from 1 µg of total RNA using Prime Script RT Master Mix. The synthesized cDNA was subjected to PCR using AccuPower PCR PreMix (Bioneer) in a PCR machine (Bio-Rad Laboratories, Hercules, CA, USA). PCR products were analyzed by agarose gel electrophoresis (FIG. 5). The primer sets used are listed in Table 1 below.

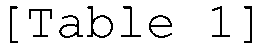

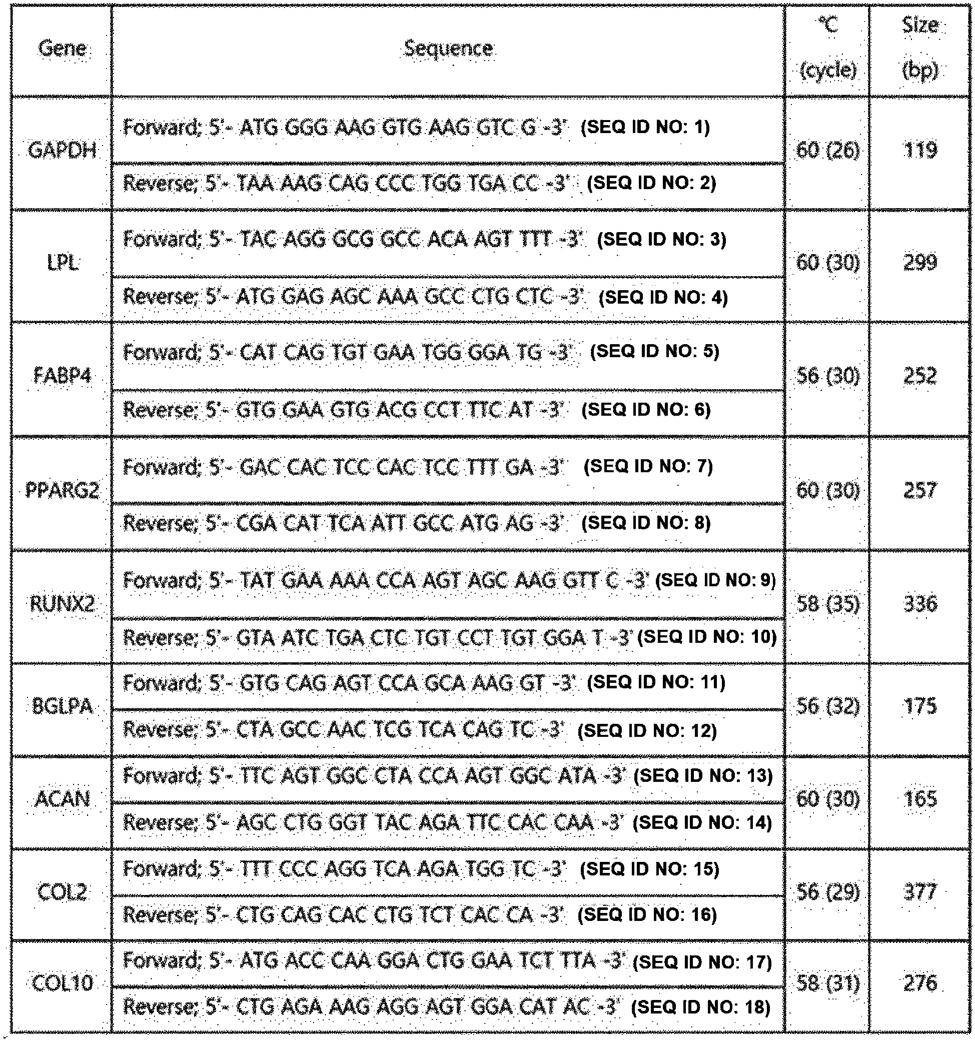

Differentiation potential was confirmed through Examples 1-3 to 1-6, and it was confirmed that the cells isolated from human adipose fat by the method of the present disclosure had very high purity, and had characteristics as stem cells even during long-term culture.

### Example 1-7. Production of Mesenchymal Stem Cell Spheroids (MiBs) through Self-Assembly of Mesenchymal Stem Cells (MSCs)

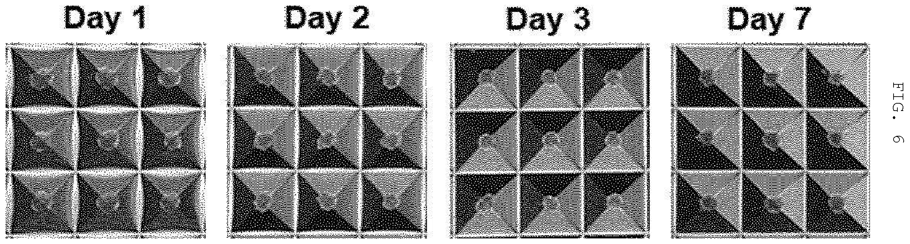

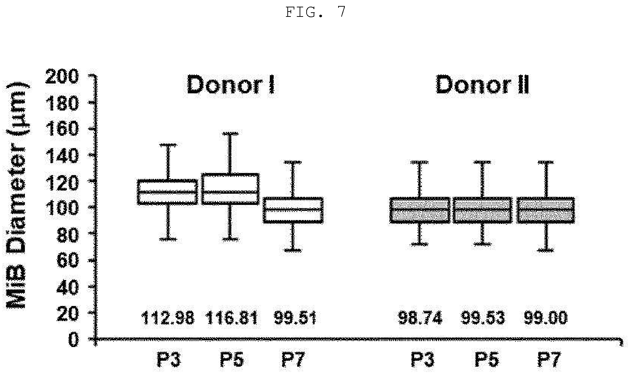

To facilitate self-assembly of MSCs, MSCs cultured in an incubator were washed with PBS and treated with proteolytic enzymes such as trypsin and collagenase to recover single cells. The cells were seeded in wells and subjected to physical stimulation via centrifugation, followed by 3D culture in a 37°C incubator for 1, 2, 3, and 7 days. Over time, mesenchymal stem cell spheroids (also referred to herein as MiBs), spherical cell structures of consistent size, were formed (FIG. 6). Mesenchymal stem cell spheroids were passed through a double-stack cell strainer with pore sizes of 70 µm and 300 µm to thereby collect uniformly sized mesenchymal stem cell spheroids. The mesenchymal stem cell spheroids were imaged using a JuLi stage system, and the MiB diameter was measured using ImageJ software. There was no significant difference in the diameter of mesenchymal stem cell spheroids depending on the donor and passage number, and mesenchymal stem cell spheroids with a diameter ranging from a minimum of 40 µm to a maximum of 260 µm were generated in each well, with an average diameter of 98.74 µm to 116.81 µm (FIG. 7).

### Example 1-8. Cell Viability of Mesenchymal Stem Cell Spheroids (MiBs) by Donor and Passage

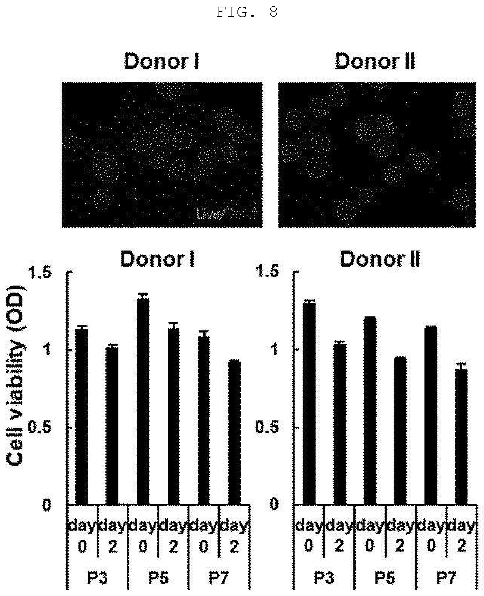

To investigate the viability of living cells within the mesenchymal stem cell spheroids, cell viability was measured using the LIVE/DEAD^{™} Viability/Cytotoxicity Kit and the MTT Cell Proliferation Assay kit. Using the LIVE/DEAD viability/cytotoxicity kit, living cells were fluorescently stained green and dead cells were fluorescently stained red. Most cells constituting the mesenchymal stem cell spheroids were viable, while cells that were poorly attached and weakly bound to the outside of the mesenchymal stem cell spheroids were stained red (FIG. 8). Compared to when cultured in two dimensions, the viable cells showed cell viability of 84% when passage 3 MSCs were used, and there was no significant change in cell viability between passages (FIG. 8). This indicates that cells existing within a three-dimensional environment survive more stably.

### Example 1-9. Analysis of DNA Content in Mesenchymal Stem Cell Spheroids (MiBs) to Determine Cell Growth

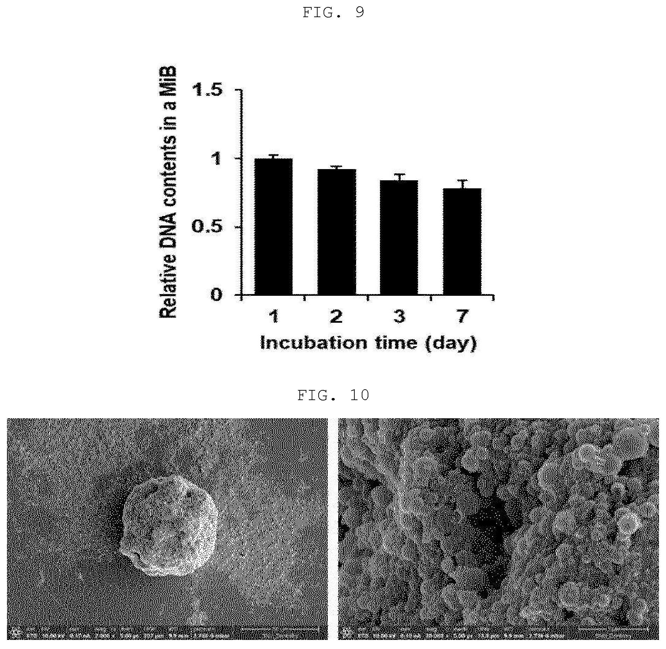

The DNA content of mesenchymal stem cell spheroids was measured using the Quant-iT^{™} PicoGreen^{™} dsDNA Assay Kit. The DNA content of the mesenchymal stem cell spheroids did not change significantly during the culture period from day 1 to day 7 (FIG. 9).

These results indicate that no significant cell proliferation was detected during the culture of mesenchymal stem cell spheroids.

### Example 1-10. Mesenchymal Stem Cell Spheroids Observed Using Scanning Electron Microscopy (SEM)

The mesenchymal stem cell spheroids were fixed and treated with an HCl-EtOH solution. The treated mesenchymal stem cell spheroid samples were post-fixed with 1% osmium tetroxide, dehydrated, and chemically dried using hexamethyldisilazane (HMDS). The samples were coated with a platinum layer using Q150TS. SEM images were acquired using Apreo S at a voltage of 10 kV. Observation of mesenchymal stem cell spheroids using SEM revealed that the mesenchymal stem cell spheroids had a highly dense spherical structure, a markedly rough surface, and numerous vesicles formed on the cell surface (FIG. 10).

### Example 1-11. Confirmation of Mesenchymal Stem Cell Characteristics of Cells Constituting the Produced Mesenchymal Stem Cell Spheroids

To determine whether the cells constituting the mesenchymal stem cell spheroids (MiBs) still retained MSC characteristics, the MSC spheroids were washed with PBS and treated with proteolytic enzymes such as trypsin and collagenase to obtain isolated single cells. The isolated cells were examined for the expression of mesenchymal stem cell surface proteins using the flow cytometry method described above.

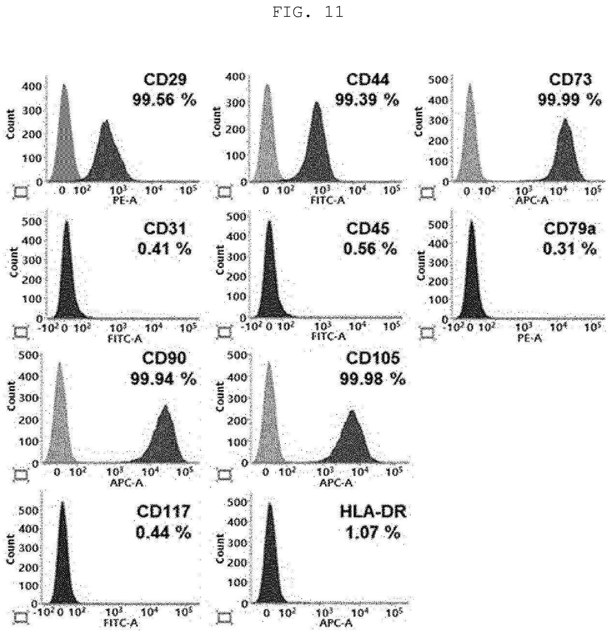

As a result of the examination, the cells constituting the mesenchymal stem cell spheroids expressed positive surface markers of MSCs such as CD29, CD44, CD73, CD90, and CD105 at 95% or more, and negative surface markers such as CD31, CD45, CD79a, CD117, and HLA-DR at 2% or less (FIG. 11).

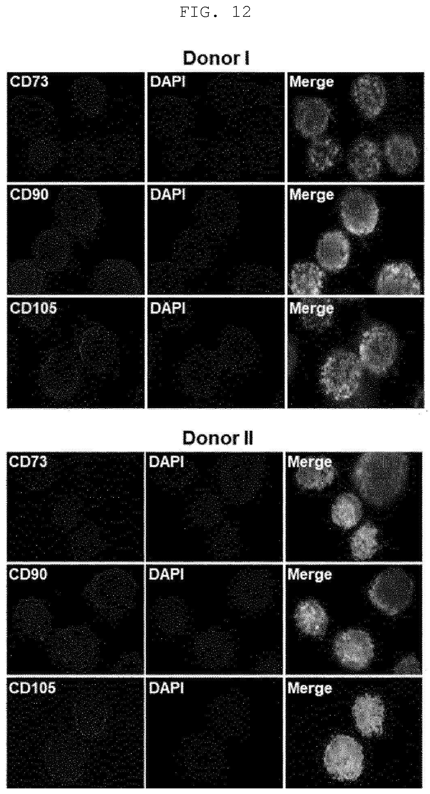

Further, mesenchymal stem cell spheroids (MiBs) were not disaggregated, but fixed with cell membrane permeability increased by using the Image-iT Fixation/Permeabilization Kit, and then cultured with antibodies against MSC markers (CD73, CD90, and CD105) in PBS containing 3% bovine serum albumin (BSA) for 18 hours at room temperature (RT). After washing with PBS, MiBs were incubated with fluorescently labeled secondary antibodies for 6 hours at room temperature. Nuclei (blue) were stained with 4',6-diamidino-2-phenylindole (DAPI) for 5 minutes at room temperature. In the fluorescently stained mesenchymal stem cell spheroids, most cells were confirmed to express the MSC markers CD73 (red), CD90 (green), and CD105 (green) (FIG. 12).

### Example 1-12. Quantitative RT-PCR Analysis of Extracellular Matrix Expression Level in Mesenchymal Stem Cell Spheroids

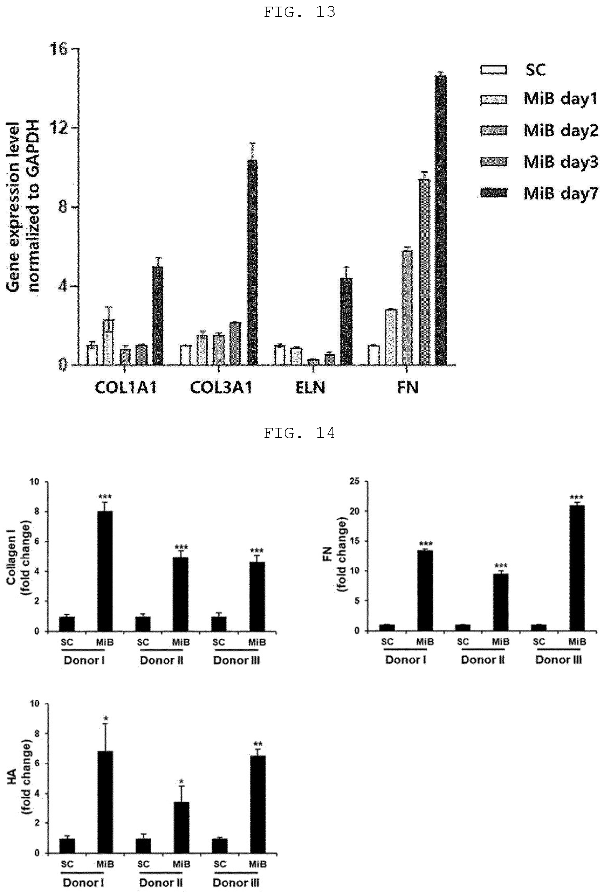

The changes in extracellular matrix gene expression when MSCs undergo morphological transformation into mesenchymal stem cell spheroids through 3D culture were investigated using quantitative real-time RT-PCR (QRT-PCR). As described in Example 1-6, RNA was isolated from mesenchymal stem cells (SCs) or mesenchymal stem cell spheroids (MiBs) cultured for 1 and 7 days using TRI reagent, and cDNA was synthesized using Prime Script RT Master Mix. PCR reactions were performed using 2X qPCRBIO SyGreen MiX Hi-ROX. QuantiTect Primer Assays (Qiagen) were used as primers for COL1A1 (collagen type I), COL3A1 (collagen type 3), ELN (Elastin), and FN1 (fibronectin). GAPDH was used as an expression level correction. The analysis results are shown in FIG. 13.

As shown in FIG. 13, Fibronectin expression increased rapidly with culture time in mesenchymal stem cell spheroids, and type 1 collagen expression also increased on day 1 of culture compared to 2D-cultured mesenchymal stem cells. Furthermore, it is expected that ECM-related gene expression increases in the mesenchymal stem cell spheroids of the present disclosure, and that ECM production is possible by MSCs within the mesenchymal stem cell spheroids even after administration for at least 7 days.

### Example 1-13. Analysis of Extracellular Matrix Expression Level in Mesenchymal Stem Cell Spheroids Using Enzyme-Linked Immunosorbent Assay

The mesenchymal stem cells or mesenchymal stem cell spheroids of the present disclosure were lysed with a mammalian protein extraction reagent containing 1 mM phenylmethanesulfonyl fluoride and a protease inhibitor cocktail. After centrifugation at 16,000 xg for 20 minutes at 4°C, type I collagen, fibronectin, and hyaluronic acid were analyzed using an enzyme-linked immunosorbent assay (ELISA) kit. Type I collagen, fibronectin, and hyaluronic acid were accumulated in mesenchymal stem cell spheroids (MiBs) (FIG. 14).

### Example 1-14. Confirmation of Collagen Expression in Mesenchymal Stem Cell Spheroids Using Fluorescent Immunostaining

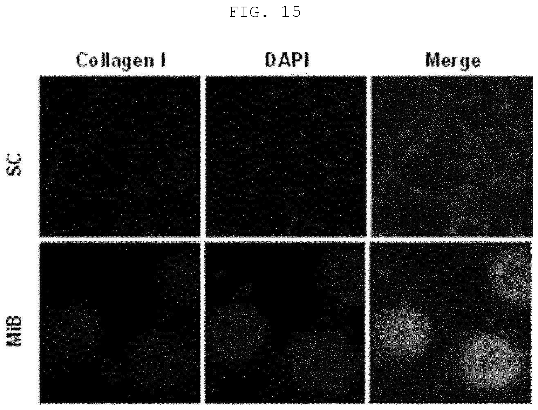

To confirm collagen expression in the mesenchymal stem cell spheroids (MiBs) of the present disclosure, paraffin-embedded mesenchymal stem cell spheroids were sectioned to 4 µm thickness to prepare slides. After deparaffinization and hydration of the slides, the mesenchymal stem cell spheroids (MiBs) were immunostained with a fluorescently labeled type 1 collagen antibody to analyze collagen expression. The results are shown in FIG. 15.

As could be confirmed in FIG. 15, the expression of type 1 collagen was found to be significantly higher in mesenchymal stem cell spheroids (MiBs) compared to single cells (FIG. 15).

### Example 1-15. Confirmation of Collagen Expression in Mesenchymal Stem Cell Spheroids Using Histological Analysis

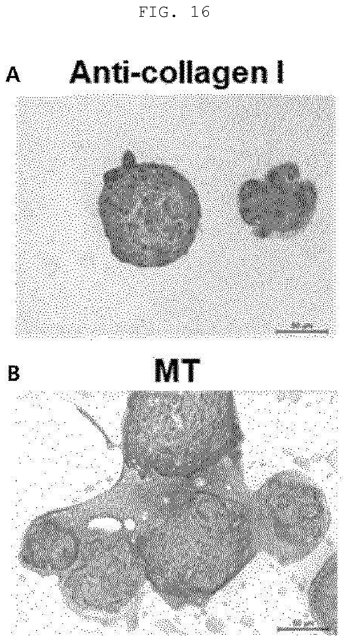

To confirm collagen expression in the mesenchymal stem cell spheroids (MiBs) of the present disclosure through histological analysis, paraffin-embedded mesenchymal stem cell spheroids were sectioned to 4 µm thickness to prepare slides as in Example 1-14. After deparaffinization and hydration, the slides were stained with immunohistochemistry (IHC) using a primary antibody against type I collagen and a biotinylated secondary antibody, and collagen was confirmed to be stained brown in the mesenchymal stem cell spheroids (FIG. 16A).

In addition, when stained using a trichrome staining kit, the blue-stained area was identified, confirming the presence of collagen in the mesenchymal stem cell spheroids (FIG. 16B).

### Example 1-16. Quantitative RT-PCR Analysis of Growth Factor Expression in Mesenchymal Stem Cell Spheroids

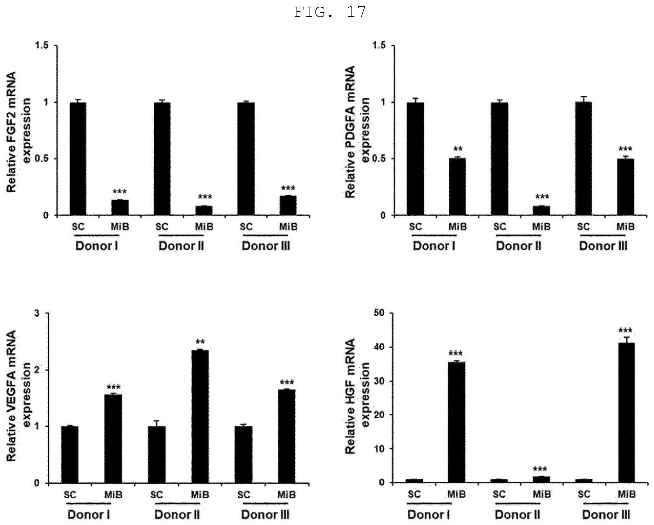

The growth factor expression of the mesenchymal stem cell spheroids (MiBs) of the present disclosure was analyzed. Specifically, quantitative RT-PCR was performed as described in Example 1-12 to investigate the gene expression of fibroblast growth factor 2 (FGF2), platelet-derived growth factor subunit A (PDGFA), vascular endothelial growth factor A (VEGFA), hepatocyte growth factor (HGF), and insulin-like growth factor 1 (IGF-1) in the mesenchymal stem cell spheroids (MiBs). Primers were designed as shown in Table 2, and quantitative RT-PCR was performed as described above, and the results are shown in FIG. 17.

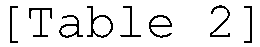

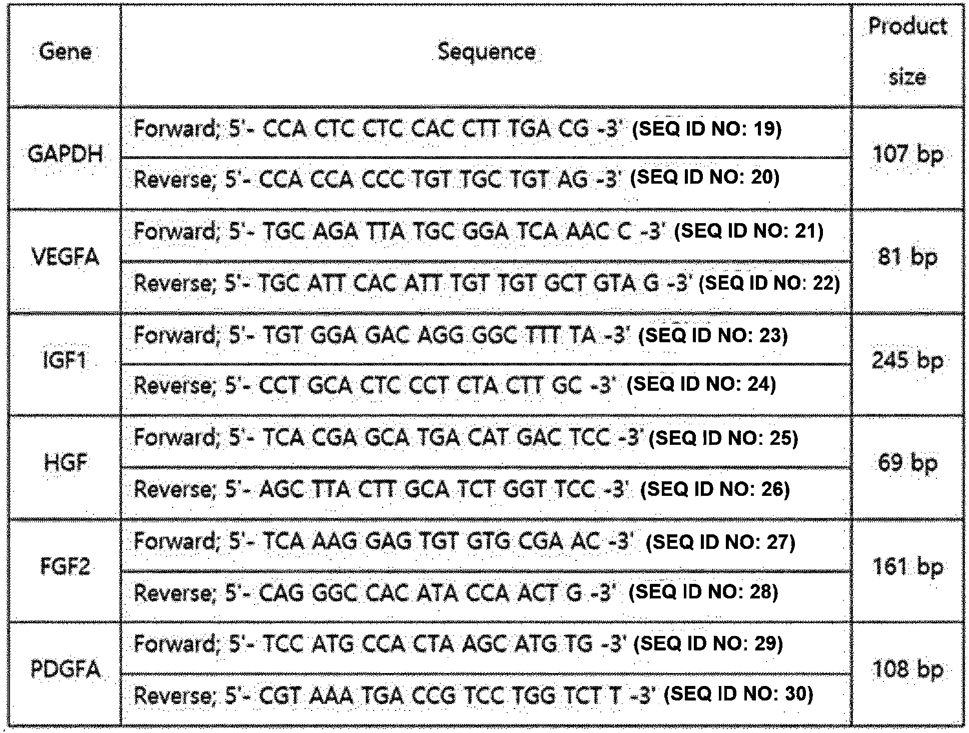

Analysis results showed that the expression of several growth factors had a positive effect on skin regeneration. As shown in FIG. 17, the expression levels of FGF2 and PDGFA in mesenchymal stem cell spheroids (MiBs) decreased, but the expression levels of VEGFA and HGF increased. This indicates that mesenchymal stem cell spheroids contribute to angiogenesis and the regeneration of damaged tissue.

### Example 2. Efficacy Testing Using Animal Models

### Example 2-1. Confirmation of Skin Regeneration, and Skin Wound and Scar Treatment Efficacy Using Wound-Induced Animal Models

### (1) Evaluation of Masson's Trichrome (MT) Collagen Production Ability

Using a scalpel, a full-thickness wound-induced model was created by making symmetrical incisions along the rat spinal midline and bluntly dissecting the subcutaneous tissue to remove the skin down to the dermal layer without damaging the muscle layer. As shown in Table 3, low (0.5 x 10⁶ cells), medium (1.0 x 10⁶ cells), and high (2.0 x 10⁶ cells) doses of TRTP-101 and excipients thereof were administered intradermally or subcutaneously, or applied topically to wound-induced rats, respectively. The wounds were protected by applying 3M's Tegaderm, and collagen area, etc., were measured on Day 21.

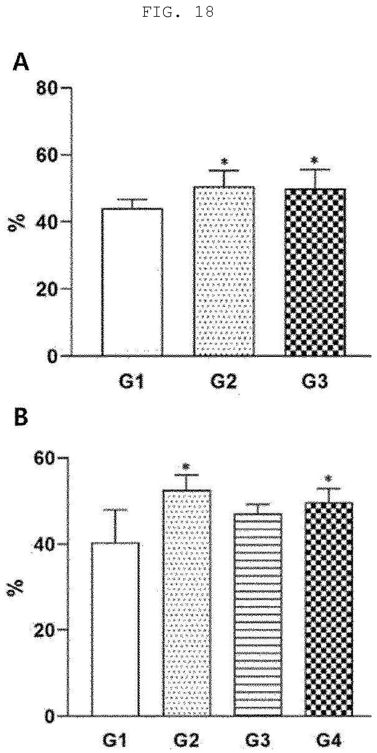

The skin tissue samples were stained with Masson's Trichrome (MT), three different images that were randomly selected from the tissue were analyzed using ZEN software to measure the collagen area in the blue region. The collagen staining area was expressed as a percentage of the total image area, and the results are shown in FIG. 18. FIG. 18A shows the results after intradermal administration, and FIG. 18B shows the results after topical application.

**[Table 3]**

| Administration Route | Administration Group | Administration Substance | Dose (cells/site) |
|---|---|---|---|
| Intradermal Administration | G1 (Control) | Vehicle (DMEM) | 0.2 mL |
| | G2 | Adipose-derived mesenchymal stem cell spheroids | 0.5 × 10⁶ (Low dose) |
| | G3 | Adipose-derived mesenchymal stem cell spheroids | 1.0 × 10⁶ (medium dose) |
| Topical Application | G1 (Control) | Vehicle (DMEM) | 0.2 mL |
| | G2 | Adipose-derived mesenchymal stem cell spheroids | 0.5 × 10⁶ (low dose) |
| | G3 | Adipose-derived mesenchymal stem cell spheroids | 1.0 × 10⁶ (medium dose) |
| | G4 | Adipose-derived mesenchymal stem cell spheroids | 2.0 × 10⁶ (high dose) |
| Subcutane ous Administr ation | G1 (Control) | Vehicle (DMEM) | 0.2 mL |
| | G2 | Adipose-derived mesenchymal stem cell spheroids | 1.0 × 10⁶ (medium dose) |

As shown in FIG. 18A, when administered intradermally, the collagen area significantly increased by 51% in the low-dose group administered with the adipose-derived mesenchymal stem cell spheroids of the present disclosure and by 50% in the medium-dose group, compared to 44% in the vehicle-administration group. Thus, it was confirmed that statistically significantly more collagen was produced in the test group administered the adipose-derived mesenchymal stem cell spheroids of the present disclosure on Day 21, the start of the remodeling phase.

Further, as can be seen in FIG. 18B, when applied topically, the collagen area significantly increased by 53% in the low-dose group administered with the adipose-derived mesenchymal stem cell spheroids of the present disclosure, by 50% in the high-dose group, and by 47% in the medium-dose group, compared to 40% in the vehicle-administration group. Therefore, compared to the control group, the test group administered the adipose-derived mesenchymal stem cell spheroids of the present disclosure showed greater collagen production.

Table 4 below summarizes the results of skin collagen amount in normal rats and in wound-induced rats after intradermal administration or topical application of the adipose-derived mesenchymal stem cell spheroids of the present disclosure. As can be seen in Table 4, when comparing the amount of collagen in normal rats with the amount of collagen after TRTP-101 administration (unit: %), it was confirmed that the adipose-derived mesenchymal stem cell spheroids of the present disclosure showed 50% collagen recovery on Day 21 after wounding, confirming a very significant tissue regeneration of up to 81% compared to the skin collagen amount of a 10-week-old normal rat of approximately 62% (Song et al.).

**[Table 4]**

| Rat | | | | | |
|---|---|---|---|---|---|
| Normal Rat Skin Collagen Amount (Song et al.) (Unit: %) | | Intradermal Administration (Unit: %) | | Topical Application (Unit: %) | |
| Week 10 | Week 54 | Control Group (Day 21) | Test Group (Day 21) | Control Group (Day 21) | Test Group (Day 21) |
| 62 | 54 | 44 | 50 | 40 | 50 |

### (2) Evaluation of Skin Regeneration and Wound Healing Following Intradermal and Subcutaneous Administration

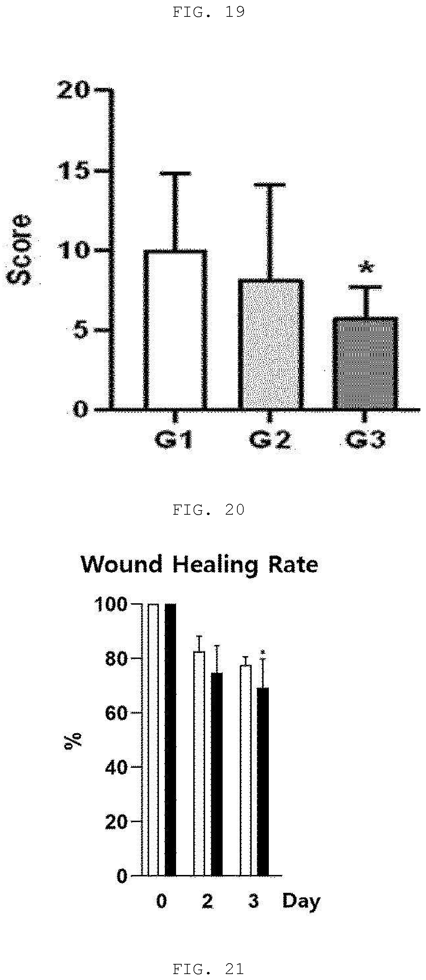

In order to evaluate the degree of skin regeneration and wound healing in the intradermal administration test, six indicators were evaluated: four indicators of inflammation level, necrosis, epithelialization, and collagenization in tissue slides stained with Hematoxylin and Eosin (H&E), and two indicators of collagen fiber density and collagen fiber arrangement in slides stained with MT. Significance was assessed by calculating the total score for each rat on a 0-5 scale, with lower scores assigned to improved skin regeneration and wound healing. The results of a comprehensive scoring of skin regeneration and wound healing indicators are shown in Table 5 below and FIG. 19. Referring to Table 5 and FIG. 19, when administered intradermally, the medium-dose group showed significant skin regeneration and wound healing compared to the vehicle-administration group.

**[Table 5]**

| | Vehicle-Administration group | Low-Dose Group (TRTP-101) | Medium-Dose Group (TRTP-101) |
|---|---|---|---|
| Average | 9.99 | 8.22 | 5.75* |
| S.E. | 1.52 | 1.85 | 0.62 |

| | | | |
|---|---|---|---|
| Each time point represents the mean + S.E. (n=10). * p<0.05, significant difference from the normal control group (G1) by Student's t-test. | | | |

In addition, in the subcutaneous administration test, the wound area ratio was analyzed to evaluate the degree of skin regeneration and wound healing, and it was observed that the wound area ratio of the TRTP-101 1.0 x 10⁶ cells/head administration group (G2) tended to be lower than that of the induced control group (G1) from Day 2 to Day 4 after administration. In particular, on Day 3, the wound area thereof was significantly reduced compared to the vehicle-administration group (FIG. 20). Based on the above results, it is believed that TRTP-101 administration to the wound site aided in the early stages of wound healing.

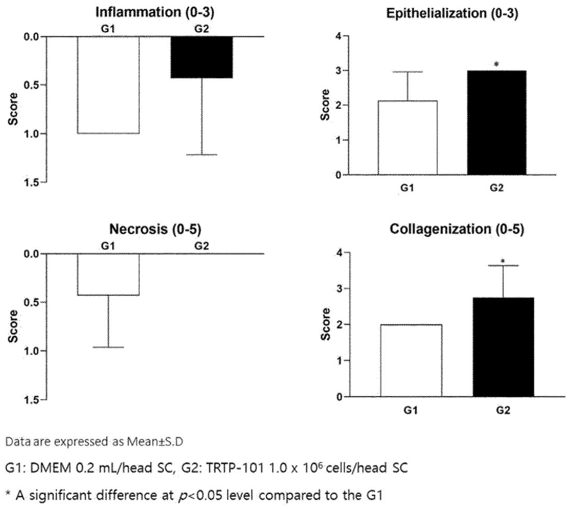

Furthermore, the histopathological examination results, which assessed and scored four indicators: inflammation, necrosis, epithelialization, and collagenization, are shown in FIG. 21. As shown in FIG. 21, the levels of inflammation and necrosis were reduced, and the TRTP-101 subcutaneous administration group (G2) showed a significant increase in epithelialization and collagen production compared to the vehicle-administration group.

### Example 2-2. Efficacy Test Using Normal Nude Mice

### (1) Analysis of Collagen Positive Area and Type 1 Collagen Positive Area According to Dose

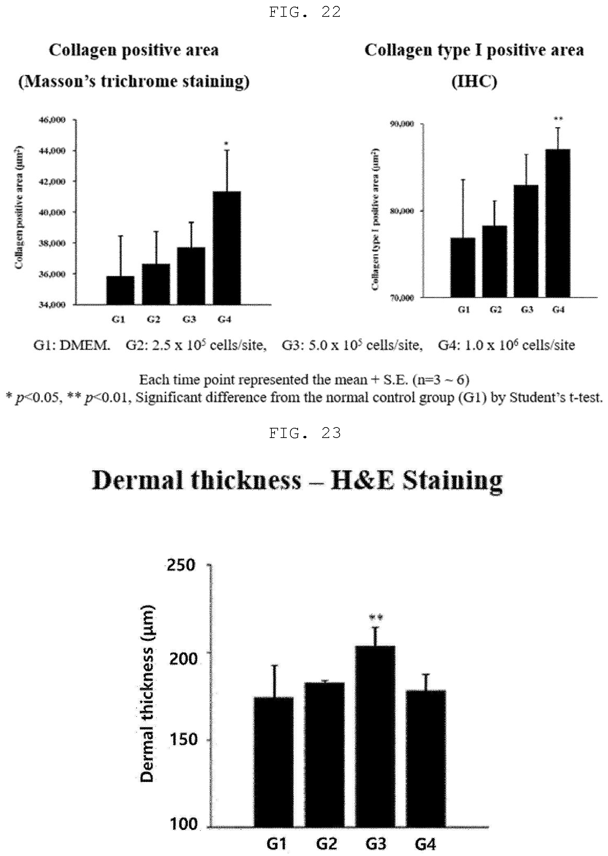

Low (0.25 x 10⁶) (G2), medium (0.5 x 10⁶) (G3), and high (1.0 x 10⁶) (G4) doses of TRTP-101 and vehicle (G1) were administered as a single intradermal injection at two sites, each at 0.1 mL/site, to the left and right dorsal regions of BALB/c nude mice, which lack mature T cells but are not completely immunosuppressed, and the in vivo persistence was evaluated. The results are shown in FIGS. 22 and 23.

As shown in FIG. 22, the synthesis of collagen and type 1 collagen, which serve as structural support, significantly increased in the test group administered the adipose-derived mesenchymal stem cell spheroids of the present disclosure compared to the control group at Week 4 after administration (each time point represents mean + S.E. (n=3-6); * p<0.05, ** p<0.01, significant difference from the vehicle-administration group by Student's t-test).

### (2) Assessment of In Vivo Persistence

As shown in FIG. 23, the dermal thickness of actual mice significantly increased compared to the control group at Week 8. In addition, the medium-dose group had the thickest dermal layer, and showed a significant increase compared to the control group.

### Example 3. Efficacy Test through In Vitro Experiment

### Example 3-1. Confirmation of ECM Production Ability

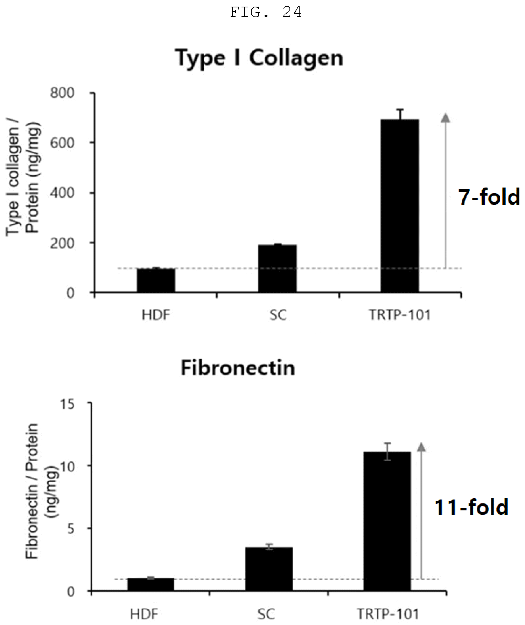

In order to confirm the degree of production of ECM-related substances as in vitro efficacy, the production ability of Type I collagen and FN of adipose-derived mesenchymal stem cell spheroids (TRTP-101) of the present disclosure was compared to the same number of Human dermal fibroblasts (HDFs) and MSC single cells (SCs) using ELISA analysis. The quantification results are shown in FIG. 24.

As shown in FIG. 24, the amount of type I collagen and FN was significantly increased in the mesenchymal stem cell spheroids of the present disclosure compared to HDFs and SCs. The expression levels of collagen type I and fibronectin, which combines ECM components to form a scaffold network, were approximately 7-fold and 11-fold higher, respectively, compared to HDF. The adipose-derived mesenchymal stem cell spheroids of the present disclosure were found to produce significantly higher levels of ECM in vitro than HDF.

These results suggest that when the adipose-derived mesenchymal stem cell spheroids of the present disclosure are applied in vivo or in humans, it is expected to have an effect of building up the volume of atrophic scars through treatment and regeneration using FN, which supports skin and connective tissue by producing Collagen Type I, the principal component of dermal tissue, and performs cell attachment, growth, and wound healing.

### Example 3-2. Confirmation of Growth Factor Secretion

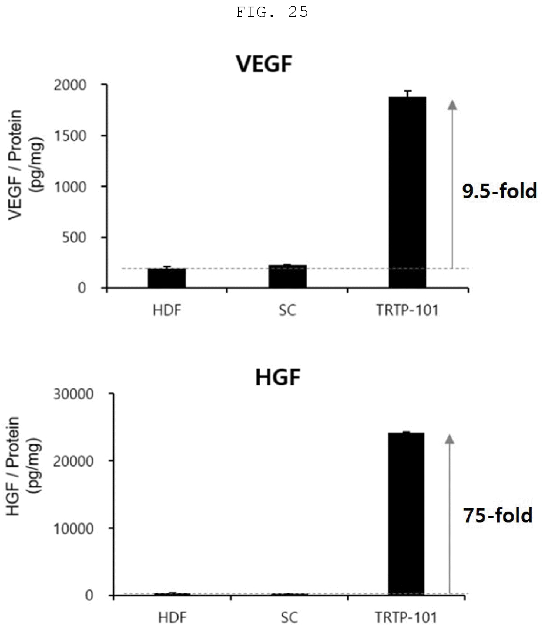

Analysis of gene expression levels of VEGFA and HGF using qRT-PCR confirmed that the adipose-derived mesenchymal stem cell spheroids of the present disclosure exhibited higher expression of these growth factors compared to human dermal fibroblasts (HDFs) and MSC single cells (SCs) in vitro. The results are shown in FIG. 25.

As shown in FIG. 25, the protein expression levels of VEGF and HGF in the adipose-derived mesenchymal stem cell spheroids of the present disclosure were approximately 9.5-fold and approximately 75-fold higher, respectively. Therefore, when the adipose-derived mesenchymal stem cell spheroid of the present disclosure was applied to an in vivo wound model, it was confirmed that the rapid recovery observed on Day 3, which is the initial stage of wound healing, was due to the high expression of growth factors related to wound healing in the adipose-derived mesenchymal stem cell spheroid of the present disclosure.

### Example 4. Tolerability, Safety, and Efficacy Study in Patients with Atrophic Scars

The efficacy of TRTP-101 (adipose-derived mesenchymal stem cell spheroids) was evaluated after a single administration in patients aged 19 years or older with atrophic scars seeking scar correction. Patients with four or more atrophic scars measuring 0.4 mm³ or greater were treated. The study subjects may include those classified as KCD disease code L90.

TRTP-101 (adipose-derived mesenchymal stem cell spheroids) was produced using fat collected from the study subjects' abdomen or thighs. The safety, tolerability, and efficacy of the spheroids were evaluated over a period of time after a single administration to the subject.

After local anesthesia with 1% lidocaine in the patient's abdomen or thigh, 60-100 mL of tumescent solution (0.9% normal saline, 2% lidocaine, 0.1% epinephrine, 8.4% sodium bicarbonate) was injected using a cannula, and 60 mL or more of fat was extracted through liposuction. Autologous adipose-derived mesenchymal stem cells (ADMSCs) were selected and mass-produced from this fat, and TRTP-101 (ADMSC spheroids) was prepared through 3D culture to a density of 0.5 ~ 4.0 × 10⁷ cells/mL.

The syringe was gently shaken to resuspend the cells in the medium, and the cells were injected intradermally using a 29 or 30 gauge needle as a single dose after disinfecting the injection site. The cells were administered to induce tautness of the atrophic scar area, with a minimum of 0.4 × 10³ cells and a maximum of 2 × 10⁶ cells per site.

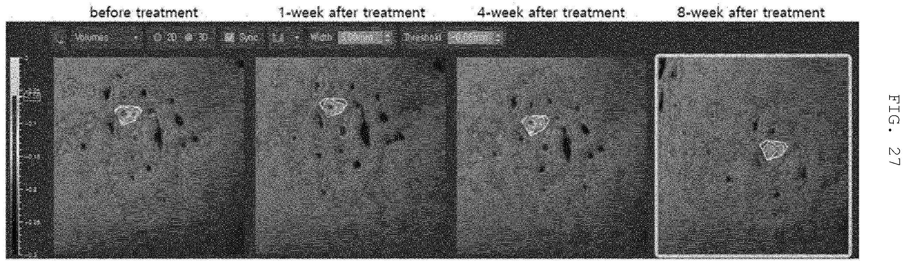

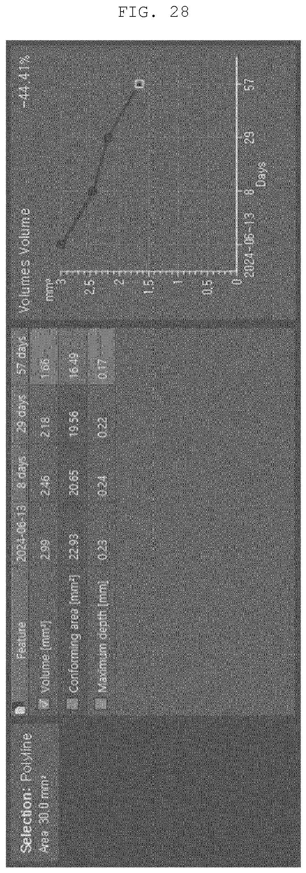

Subjects who could obtain data on exploratory efficacy endpoints after administration of the clinical trial drug were selected, and exploratory efficacy was analyzed. Using Antera 3D^{®}, images of the scar area were obtained before and after administration of TRTP-101 (adipose-derived mesenchymal stem cell spheroids) (FIG. 27), and the volume of the atrophic scar (volume of depression) was measured. The results showed a decrease in the volume of the atrophic scar (FIG. 28). To document the site of administration of the clinical trial drug, images of the administration site were captured and stored along with the relevant supporting documentation. The pre- and post-administration changes in the administration site of the clinical trial drug were determined by using the atrophic scar volume measured at each time point.

Referring to FIG. 28, the scar size significantly decreased 8 weeks after administration compared to before administration.

As a result of the tolerability and safety evaluation, no dose-limiting toxicity (DLT) corresponding to an adverse drug reaction (ADR) of Grade 3 or higher according to Common Terminology Criteria for Adverse Events (CTCAE) Ver. 5.0 occurred during the 4-week period following TRTP-101 administration. All adverse reactions were classified and analyzed by the latest version of MedDRA's System Organ Class (SOC) and Preferred Term (PT).

The clinical trial results of Example 4, consistent with the efficacy test using an animal model, demonstrated significant or remarkable efficacy in the treatment of atrophic scars. Specifically, in the TRTP-101 administration group, which is a pharmaceutical composition comprising adipose-derived mesenchymal stem cell spheroids of the present disclosure, atrophic scar volume was significantly reduced to a degree indicating therapeutic efficacy.

### Example 5. Physical Characterization of Mesenchymal Stem Cell Spheroids (MiBs) of the Present disclosure

### (1) Wet Weight Analysis

Wet weight analysis was performed to determine the characteristics of the mesenchymal stem cell spheroids (referred to as MiBs or TRTP-101 in the present disclosure) produced in vitro according to Example 1. Specifically, to determine the weight of the mesenchymal stem cell spheroids of the present disclosure, the combined weight of 1,200 spheroids was measured to estimate the individual weight. The results are shown in Table 6 below.

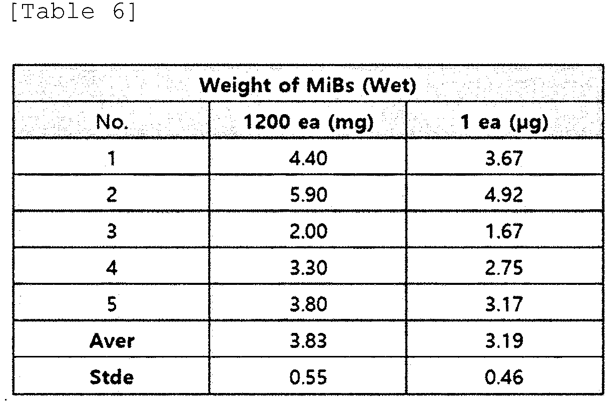

Measurement results confirmed that the wet weight of the mesenchymal stem cell spheroids according to the present disclosure was approximately 3.19±0.46 µg per spheroid.

### (2) Dry Weight Analysis

To accurately measure the weight of the mesenchymal stem cell spheroids according to the present disclosure, excluding the influence of solvents such as medium or buffer, an experiment was conducted to measure dry weight using a freeze-drying method.

### ① Workflow of Dry Weight Measurement Experiment

The following is the workflow for the experiment conducted to determine dry weight, a characteristic of mesenchymal stem cell spheroids (MiBs) in vitro. The mesenchymal stem cell spheroids (MiBs) according to the present disclosure were tested separately by two experimenters to determine inter-experimenter variation. Cells were harvested at 70% confluency, and 0.5K mesenchymal stem cell spheroids (MiBs) were cultured in a 5% CO₂ incubator at 37°C for 2 days, supplemented with DMEM containing 10% fetal bovine serum and 1X antibiotic and antimycotic. The cells were grouped as shown in Table 7 below.

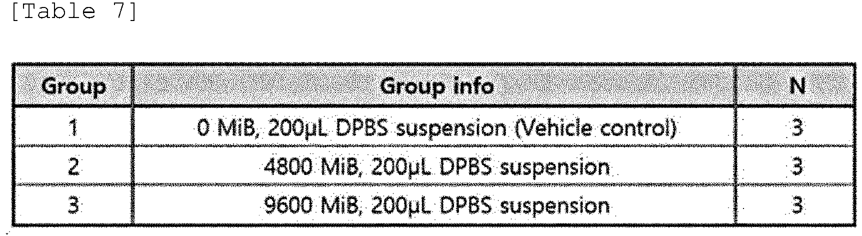

During the mesenchymal stem cell spheroid (MiB) culture period, 5 mL tubes and parafilm used for harvesting were weighed prior to harvesting using an electronic balance. Each group was weighed three times using an electronic balance, and the average of the three measurements was calculated. Before each measurement, the electronic balance was tared (reset to zero). If a weight difference of 0.2 µg or more was detected from the previous weight, the balance was reset to zero and the weight was measured again.

Mesenchymal stem cell spheroids (MiBs) cultured for 48 hours from the time of production were collected in 5 mL tubes in triplicate, each containing 0, 4,800, and 9,600, and then suspended in 200 µL of DPBS. The 5 mL tubes were sealed with parafilm, punctured with a 30-gauge needle, and dried in a freeze-dryer for 2 days. The freeze-dried samples were weighed three times using an electronic balance and the average of the measured values was calculated. The dry weights of 4,800 and 9,600 mesenchymal stem cell spheroids (MiBs) were determined by subtracting the weights of the pre-measured 5 mL tube and parafilm from the weight of the freeze-dried sample. The calculated dry weight was then divided by the number of MiBs to obtain the weight of one MiB.

### ② Dry Weight Measurement Results of Mesenchymal Stem Cell Spheroids (MiBs) and Singlet Mesenchymal Stem Cell Spheroids (MiBs)

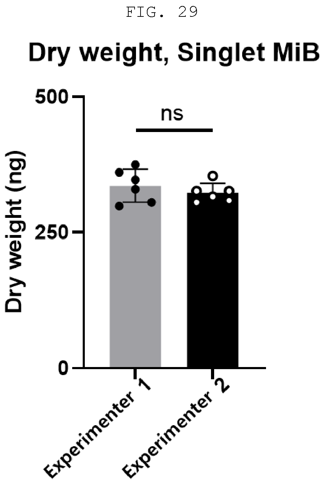

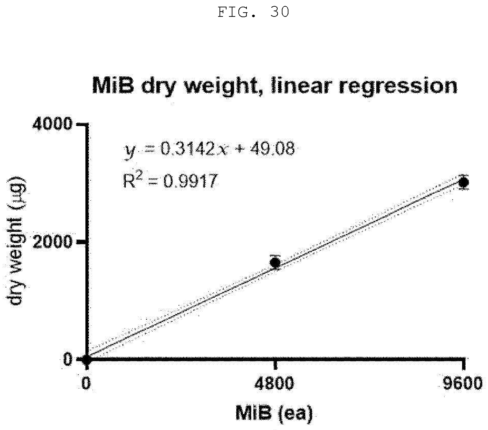

The dry weight of 4,800 and 9,600 mesenchymal stem cell spheroids (MiBs) and singlet mesenchymal stem cell spheroids (MiBs), measured according to the dry weight measurement experiment workflow, are shown in Table 8. The variation in singlet mesenchymal stem cell spheroid (MiB) dry weight measurements between the two experimenters is shown in FIG. 29, and the linear trend results for the weight of mesenchymal stem cell spheroids (MiBs) are shown in FIG. 30.

**[Table 8]**

| Weight of MiBs | | | |
|---|---|---|---|
| Experimenter | Group | MiB Dry Weight (µg) | Dry Weight of 1 MiB (ng) |
| Experimenter 1 | 1 | 0 | 0 |
| | | 0 | 0 |
| | | 0 | 0 |
| | 2 | 1666.7 | 347.2 |
| | | 1733.3 | 361.1 |
| | | 1800.0 | 375.0 |
| | 3 | 2933.3 | 305.6 |
| | | 3166.7 | 329.9 |
| | | 2866.7 | 298.6 |
| Experimenter 2 | 1 | 0 | 0 |
| | | 0 | 0 |
| | | 0 | 0 |
| | 2 | 1700.0 | 354.2 |
| | | 1566.7 | 326.4 |
| | | 1466.7 | 305.5 |
| | 3 | 3133.3 | 326.4 |
| | | 2966.7 | 309.0 |
| | | 3033.3 | 316.0 |

Referring to Table 8 and FIG. 29, the weights of the mesenchymal stem cell spheroids (MiBs) of the present disclosure measured by Experimenters 1 and 2 were 336.2±30.5 ng and 322.9.2±17.6 ng (Mean ± S.D.), respectively, with no statistically significant differences.

Furthermore, the dry weight data of 0, 4,800, and 9,600 mesenchymal stem cell spheroids from both Experimenters were integrated and used to derive a linear trend line. The R² value was 0.9917, indicating statistical significance for this experiment (FIG. 30).

In addition, the dry weight of one mesenchymal stem cell spheroid (MiB), 317 ng, calculated from the trend line, fell within the weight range of the measured mesenchymal stem cell spheroid (MiB) in Table 8.

### Example 6. Proteomic Analysis of Mesenchymal Stem Cell Spheroids (MiBs) of Present disclosure

### (1) Analysis of Protein Counts Constituting Single Cells and Mesenchymal Stem Cell Spheroids of Present disclosure

To determine the characteristics of mesenchymal stem cell spheroids of the present disclosure compared to single cells, proteomic analysis of the produced mesenchymal stem cell spheroids and single cells was conducted.

For this analysis, protein extraction was performed by adding samples in lysis buffer containing 4% SDS, boiling at 100°C for 10 minutes, followed by centrifugation at 18,000 rpm for 10 minutes, according to the method of Lee, Jangho, et al. The proteins were measured using the bicinchoninic acid assay (BCA) and separated on an SDS-PAGE gel for in-gel digestion. The gel was fractionated by molecular weight according to the method of Lee, Sang-Yeop, et al., and washed with a solution containing 30% methanol, 10 mM ammonium bicarbonate, and 50% acetonitrile. After washing, the gel was dried and reduced with 10 mM dithiothreitol and 55 mM iodoacetamide, followed by tryptic digestion with 50 mM ammonium bicarbonate at 37°C for 12-16 hours. Tryptic peptides were extracted with a 50 mM ammonium bicarbonate solution containing 5% trifluoroacetylacid (TFA) and 50% acetonitrile and stored at 4°C.

The solution stored according to the above method was dissolved in 0.5% TFA and analyzed by liquid chromatography with tandem mass spectrometry (LC-MS/MS). 5 µL of the eluted sample was eluted with 5%-40% acetonitrile at a flow rate of 300 nL/min for 95 or more minutes using a 100 µm × 2 cm nanoviper trap column and a 75 µm × 15 cm nanoviper analysis column (Thermo Fisher Scientific). A11 MS and MS/MS spectral data were analyzed using a QExactive Plus mass spectrometer (Thermo Fisher Scientific).

Protein identification was performed based on monoisotopic mass selection with a precursor mass tolerance of ±5 Da, a fragment mass tolerance of ±0.8 Da, up to two missed cleavages, and a modified carbamidomethylcysteine. Individual spectra were accepted based on a Mascot ion threshold score of 0.05, calculated specifically for each database search. Peptides and proteins exceeding the threshold were matched and analyzed using the Peptide Validator, with a false discovery rate (FDR) of 1.0%.

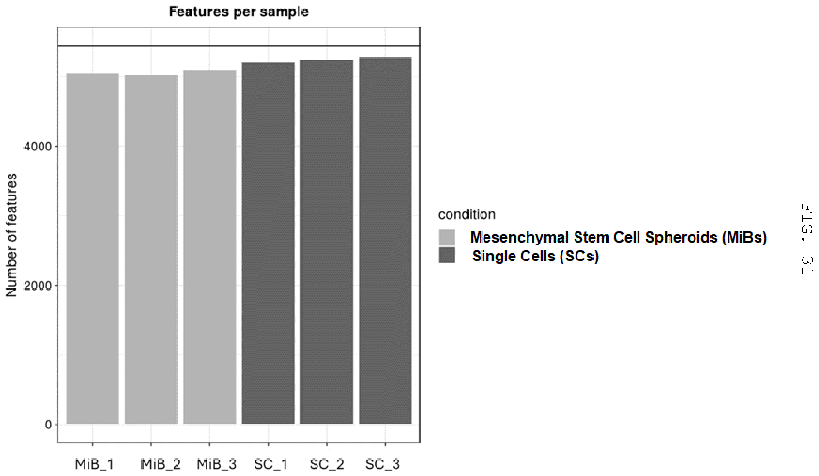

Experiments were conducted with three samples per group for single cells and the mesenchymal stem cell spheroids (MiBs) of the present disclosure, and the results are shown in FIG. 31.

As shown in FIG. 31, the analysis of the number of proteins constituting the single cells and the mesenchymal stem cell spheroids (MiBs) of the present disclosure revealed a slight increase of approximately 3% in the total number of proteins constituting each mesenchymal stem cell spheroid (MiB) and single cell.

### (2) Principal Component Analysis of Single Cells and Mesenchymal Stem Cell Spheroids of Present disclosure

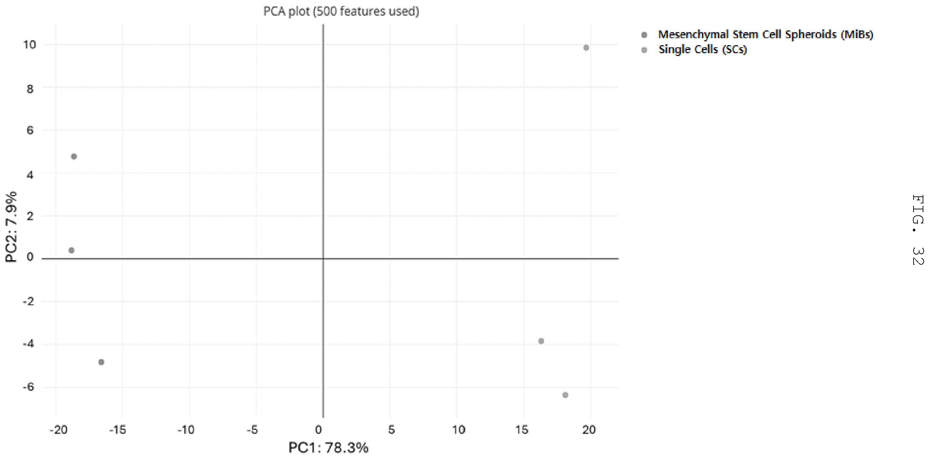

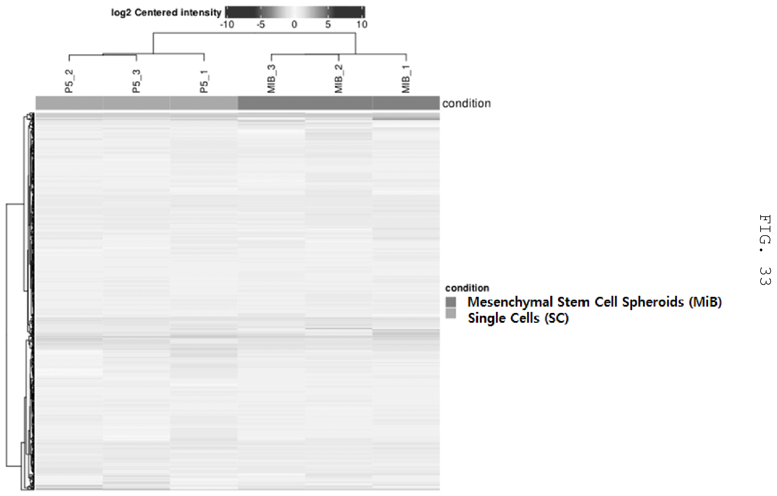

To identify intergroup correlations and differences in protein composition using the proteins constituting the samples, principal component analysis (PCA) and heatmap analysis were performed. The PCA analysis distribution graph, which shows the correlation between the proteins constituting the samples, revealed that single cells and mesenchymal stem cell spheroids were arranged in opposite positions, demonstrating a clear difference in the types of proteins they comprised. The results are shown in FIG. 32. Further, the heatmap analysis, which displays correlations between groups in rows, columns, and colors, also confirmed differences in protein composition and content between single cells and mesenchymal stem cell spheroids. The results are shown in FIG. 33.

### (3) Analysis of Protein Increase/Decrease Distribution of Mesenchymal Stem Cell Spheroids of Present disclosure

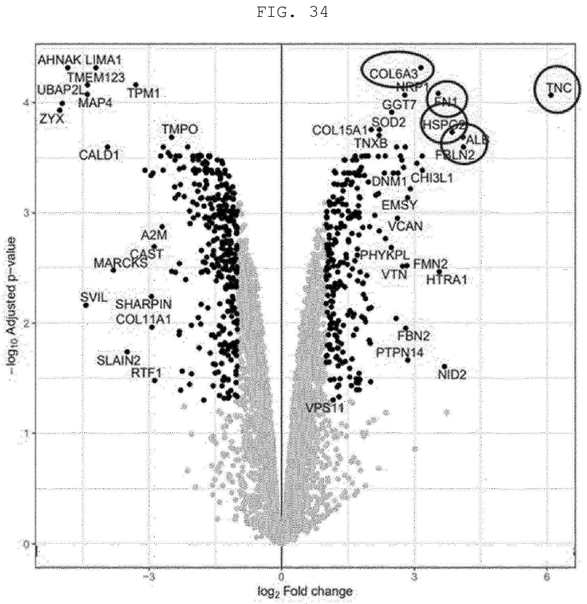

The protein increase/decrease distribution of the mesenchymal stem cell spheroids (MiBs) of the present disclosure was analyzed compared to single cells. The protein increase/decrease distribution is shown in FIG. 34, and the structural proteins increased in the mesenchymal stem cell spheroids (MiBs) of the present disclosure compared to single cells are confirmed in Table 9 below.

**[Table 9]**

| Genes | Increase (Fold Change) in Mesenchymal Stem Cell Spheroids Compared to Single Cells |
|---|---|
| Tenascin (TNC) | 68 |
| Fibulin-2 (FBLN2) | 17 |
| Basement membrane-specific heparan sulfate proteoglycan core protein (HSPG2) | 14 |
| Fibulin-1 (FBLN1) | 13 |
| Fibronectin (FN1) | 11 |
| Collagen alpha-3 (VI) chain (COL6A3) | 8 |

The analysis results of the Volcano plot (FIG. 34), which visually shows the statistical significance of changes in protein composition, showed that Tenascin (TNC) exhibited the greatest change in the mesenchymal stem cell spheroids (MiBs) of the present disclosure compared to single cells. Tenascin is a protein expressed in response to tissue damage or inflammation, and is known to promote healing by participating in cell proliferation responses through activation of the epidermal growth factor receptor (EGFR) and inflammatory signaling pathways.

Similarly, fibronectin (FN1) was one of the proteins with a significant increase. Fibronectin is known to play a key role in wound healing and tissue remodeling processes, participating in cell adhesion, growth, migration, and differentiation.

As described above, the proteins increased in the mesenchymal stem cell spheroids (MiBs) of the present disclosure compared to single cells may be functionally categorized into structural proteins, enzymes, ribosomal proteins, histone proteins, signaling proteins, and proteins related to apoptosis and differentiation. In particular, proteins related to structure were increased, which is believed to be due to the induction of self-assembly, the mechanism of formation of the mesenchymal stem cell spheroids (MiBs) of the present disclosure.

Furthermore, proteoglycan-4 (PRG4) was found to be a protein specifically expressed only in mesenchymal stem cell spheroid (MiB). PRG4 is known to regulate molecular pathways associated with wound healing, thereby inducing adipocytes at the wound site, thereby reducing fibrosis and enhancing angiogenesis to promote wound healing.

Furthermore, proteins that provide structural support in tissues such as skin, including COL5A3 (Collagen Type V Alpha 3 Chain), COL7A1 (Collagen Type VII Alpha 1 Chain), EVPL (Envoplakin), EFEMP2 (Fibulin-4), and MFAP2 (Microfibril Associated Protein 2), etc., were found to be newly produced in mesenchymal stem cell spheroids (MiBs) compared to single cells.

It was confirmed through this protein analysis that several structural proteins were specifically expressed in the mesenchymal stem cell spheroids (MiBs) of the present disclosure, and that extracellular matrix proteins also increased.

## Claims

1. A pharmaceutical composition for treating skin wounds or scars, comprising a therapeutically effective amount of mesenchymal stem cell spheroids applied topically to a target site or administered subcutaneously or intradermally.

2. The pharmaceutical composition of claim 1, wherein the mesenchymal stem cell spheroid is a three-dimensional cell aggregate of mesenchymal stem cells.

3. The pharmaceutical composition of claim 1, wherein a diameter of the mesenchymal stem cell spheroid is 10 to 800 µm.

4. The pharmaceutical composition of claim 1, wherein a wet weight of the mesenchymal stem cell spheroid is 1 to 6 µg.

5. The pharmaceutical composition of claim 1, wherein a dry weight of the mesenchymal stem cell spheroid is 250 to 500 ng.

6. The pharmaceutical composition of claim 1, wherein the mesenchymal stem cells are any one selected from the group consisting of adipose-derived mesenchymal stem cells, bone marrow-derived mesenchymal stem cells, umbilical cord blood-derived mesenchymal stem cells, umbilical cord-derived mesenchymal stem cells, and peripheral blood-derived mesenchymal stem cells.

7. The pharmaceutical composition of claim 1, wherein the mesenchymal stem cell spheroid comprises mesenchymal stem cells (MSCs) and extracellular matrix (ECM).

8. The pharmaceutical composition of claim 7, wherein the extracellular matrix comprises any one or more selected from the group consisting of collagen, fibronectin, hyaluronic acid (HA), elastin (EL), and glycosaminoglycan (GAG).

9. The pharmaceutical composition of claim 8, wherein the collagen is type 1 collagen.

10. The pharmaceutical composition of claim 1, wherein the mesenchymal stem cell spheroid exhibits increased expression of any one or more growth factors selected from the group consisting of fibroblast growth factor 2 (FGF2), platelet-derived growth factor subunit A (PDGFA), vascular endothelial growth factor A (VEGFA), hepatocyte growth factor (HGF), and insulin-like growth factor 1 (IGF-1).

11. The pharmaceutical composition of claim 1, wherein the mesenchymal stem cell spheroid comprises cells that are positive for expression of any one or more markers selected from the group consisting of CD29, CD44, CD73, CD90, and CD105 by at least 85%, 86%, 87%, 88%, 89%, 90%, 91%, 92%, 93%, 94%, 95%, 96%, 97%, 98%, or 99% or more.

12. The pharmaceutical composition of claim 1, wherein the mesenchymal stem cell spheroid comprises cells that are negative for expression of any one or more markers selected from the group consisting of CD31, CD45, CD79a, CD117 and HLA-DR by at least 10%, 9%, 8%, 7%, 6%, 5%, 3%, 2% or less.

13. The pharmaceutical composition of claim 1, wherein the mesenchymal stem cell spheroid comprises cells that are positive for expression of CD29, CD44, CD73, CD90, and CD105 by at least 85%, 86%, 87%, 88%, 89%, 90%, 91%, 92%, 93%, 94%, 95%, 96%, 97%, 98%, or 99% or more, and that are negative for expression of CD31, CD45, CD79a, CD117, and HLA-DR by at least 10%, 9%, 8%, 7%, 6%, 5%, 3%, 2% or less.

14. The pharmaceutical composition of claim 1, wherein the mesenchymal stem cell spheroid expresses any one or more proteins selected from the group consisting of Tenascin (TNC), Fibronectin (FN1), Proteoglycan-4 (PRG4), COL5A3 (Collagen Type V Alpha 3 Chain), COL7A1 (Collagen Type VII Alpha 1 Chain), EVPL (Envoplakin), EFEMP2 (Fibulin-4), MFAP2 (Microfibril Associated Protein 2), FBLN2 (Fibulin-2), HSPG2, FBLN1 (Fibulin-1), and COL6A3 (Collagen alpha-3(VI) chain).

15. The pharmaceutical composition of claim 1, wherein the pharmaceutical composition is administered once, twice or three times.

16. The pharmaceutical composition of claim 1, wherein the administration is direct administration to the target site that requires skin regeneration through collagen production.

17. The pharmaceutical composition of claim 1, wherein the target site includes a part where the dermis layer of the skin is damaged or depressed.

18. The pharmaceutical composition of claim 1, wherein the target site includes a part where the subcutaneous layer of the skin is damaged or depressed.

19. The pharmaceutical composition of claim 1, wherein the mesenchymal stem cell spheroid regenerates the skin by inducing dermal recovery within the skin.

20. The pharmaceutical composition of claim 1, wherein the mesenchymal stem cell spheroid regenerates the skin by inducing subcutaneous recovery within the skin.

21. The pharmaceutical composition of claim 1, wherein the pharmaceutical composition is in any one dosage form from the group consisting of an injection, infusion, spray, liquid, suspension, external use or patch.

22. The pharmaceutical composition of claim 1, wherein the mesenchymal stem cell spheroids are contained in an amount of 1 to 2 × 10⁵ per unit dosage.

23. The pharmaceutical composition of claim 1, wherein the scar is any one or more selected from the group consisting of bedsore scars, burn scars, hair loss scars, linear scleroderma scars, wound scars, diabetic foot ulcer scars, atrophic scars, and stretch marks.

24. The pharmaceutical composition of claim 1, wherein the wound is a wound in which the epidermis; dermis; subcutaneous; epidermis and dermis; dermis and subcutaneous; or epidermis, dermis and subcutaneous layers of the skin are damaged.

25. The pharmaceutical composition of claim 1, wherein the wound is any one or more selected from the group consisting of cuts, incisions, wounds, abrasions, contusions, penetrating wounds, incised wounds, avulsion injuries, lacerations, fractures, burns, and amputations.
